# EUROPEAN PATENT APPLICATION

(11) **EP 4 656 722 A1**
(43) Date of publication of application: **03.12.2025**
(21) Application number: 24382574.2
(22) Date of filing: 29.05.2024
(51) Int. Cl.: C12N 15/115

(54) **OR2AT4 RECEPTOR AGONIST APTAMERS AND USES THEREOF**

(71) Applicant: Fundacion Instituto De Investigacion Sanitaria Fundacion Jimenez Diaz, 28040 Madrid (ES); Universidad Carlos III de Madrid, 28919 Leganes (Madrid) (ES); Mediteknia Skin & Hair LAB SL, 35004 Las Palmas de Gran Canaria (ES); Centro de Investigaciones Energéticas, Medioambientales y Tecnológicas, 28040 Madrid (ES); Fundación para la Investigación Biomédica del Hospital Universitario Ramón y Cajal, 28034 Madrid (ES)
(72) Inventor: Martín Palma, Elena, Madrid (ES); González Muñoz, Víctor Manuel, Madrid (ES); Jiménez Acosta, Francisco, Las Palmas (ES); Carretero Trillo, Marta, Madrid (ES); Mataix Rodríguez, Manuel, Madrid (ES); Larcher Laguzzi, Fernando, Madrid (ES); de Arriba Pérez, María del Carmen, Madrid (ES); del Río Nechaevsky, Marcela Andrea, Madrid (ES)
(74) Representative: Pons IP

(57) **Abstract**

The present invention relates to OR2AT4 receptor agonist aptamers and uses thereof. The present invention relates to a nucleic acid aptamer or a fragment thereof capable of binding specifically to the OR2AT4 receptor and activating said OR2AT4 receptor, comprising a nucleotide sequence with a sequence identity of at least 80% with the sequence SEQ ID NO: 1, SEQ ID NO: 2, SEQ ID NO: 3, SEQ ID NO: 4, or SEQ ID NO: 8.

## Description

The present invention relates to OR2AT4 receptor agonist aptamers, as well as the therapeutic and cosmetic uses thereof. Therefore, the invention could be comprised in the field of biomedicine.

### STATE OF THE ART

Olfactory receptors (ORs) are expressed in the nasal epithelium, although there is increasing evidence of their ectopic expression in non-chemosensory tissues, such as prostate, muscle, liver, lung, kidney, gastrointestinal tract, spleen, pancreas, placenta, brain, and skin (Oh, S. J. (2018). Genomics & informatics, 16(1), 2.). Specifically, the OR2AT4 (olfactory receptor family 2 subfamily AT member 4) receptor has been shown to be expressed in keratinocytes, dendritic cells, and melanocytes of the skin. Certain synthetic OR2AT4 agonists, such as the odorants Sandalore and Brahmanol, are capable of activating the OR2AT4 receptor expressed in keratinocytes, demonstrating the role played by this receptor in skin wound healing processes (Busse, D., et al., (2014). Journal of investigative dermatology, 134(11), 2823-2832.). OR2AT4 expression has also been detected in the lymphoblasts of patients with acute myeloid leukaemia ( AML). Stimulation with agonists of the receptor inhibits the proliferation and increases the apoptosis of these cells, so OR2AT4 could be an interesting therapeutic target for AML (Manteniotis, S., et al.,(2016). Cell death discovery, 2(1), 1-10.). The epithelium of human hair follicles, in particular the outer root sheath, expresses OR2AT4. Stimulation of the receptor by means of the synthetic odorant Sandalore promotes hair growth by reducing apoptosis and increasing the production of the growth factor IGF-1 (Insulin-like growth factor 1), thereby prolonging the anagen phase and delaying entry into the catagen phase (Chéret, J., et al., (2018). Nature communications, 9(1), 3624.). In addition to these effects observed *ex vivo,* a randomised, double-blinded, placebo-controlled clinical trial has been conducted to demonstrate the effects of Sandalore in 60 patients with telogen effluvium. Treatment with a solution containing 1% Sandalore applied for 24 weeks resulted in an improvement in the following parameters: degree of hair loss, hair volume, terminal/vellous hair ratio, anagen/catagen-telogen ratio, as well as the patient's self-esteem (Jimenez et al., 2021. J Cosmet Dermatol 2021;20(3):784-91). Therefore, these results point to OR2AT4 as an interesting therapeutic target to promote hair growth and/or inhibit or delay hair loss in humans.

Therefore, there is a need to provide new OR2AT4 receptor agonists which can be used in the field of biomedicine and cosmetics, given the involvement of said receptor in different pathologies or conditions.

### DESCRIPTION OF THE INVENTION

The authors of the present invention have developed nucleic acid aptamers which act as OR2AT4 (olfactory receptor family 2 subfamily AT member 4) receptor agonist molecules.

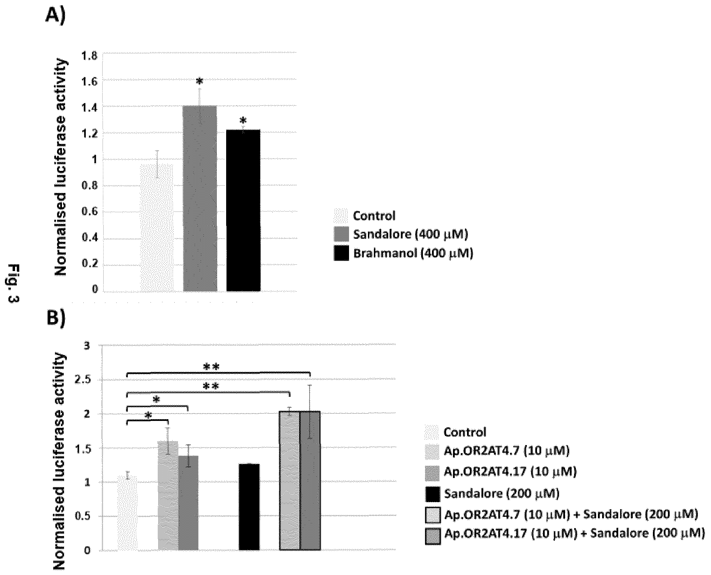

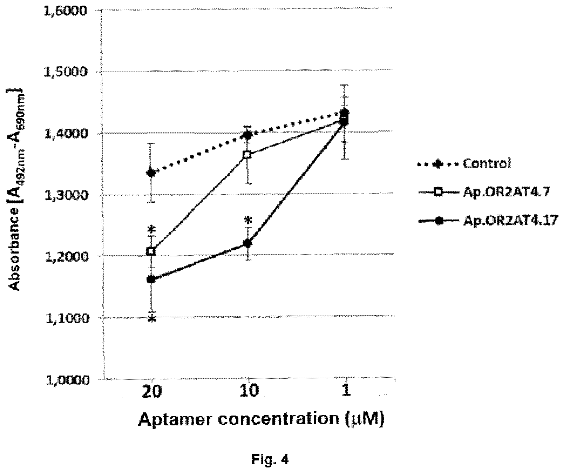

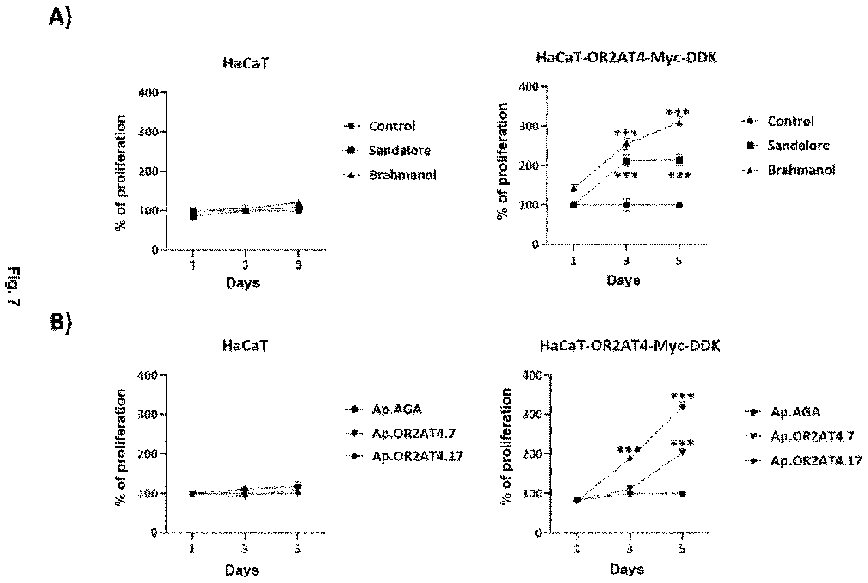

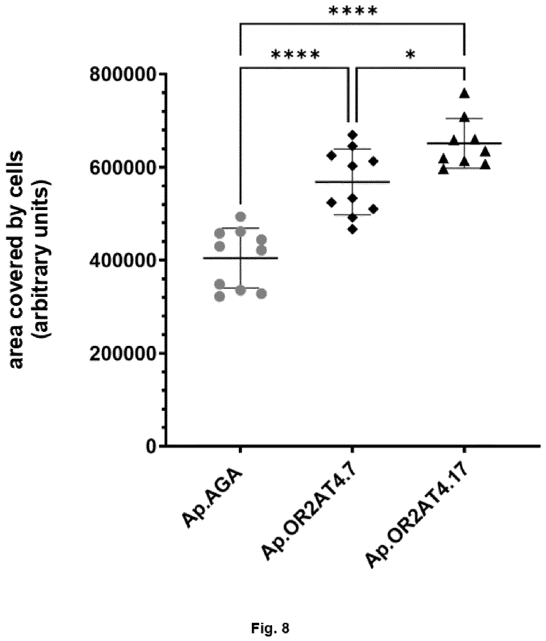

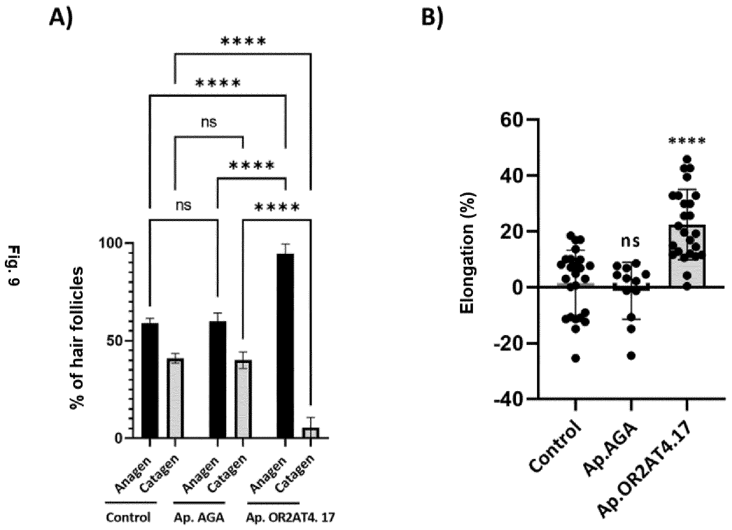

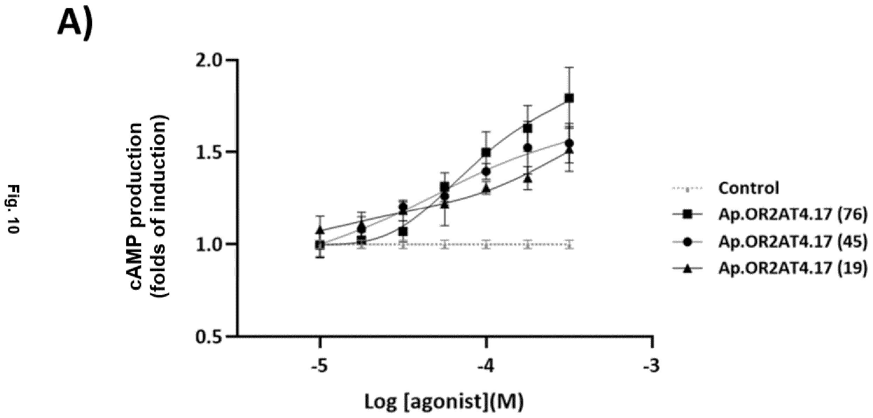

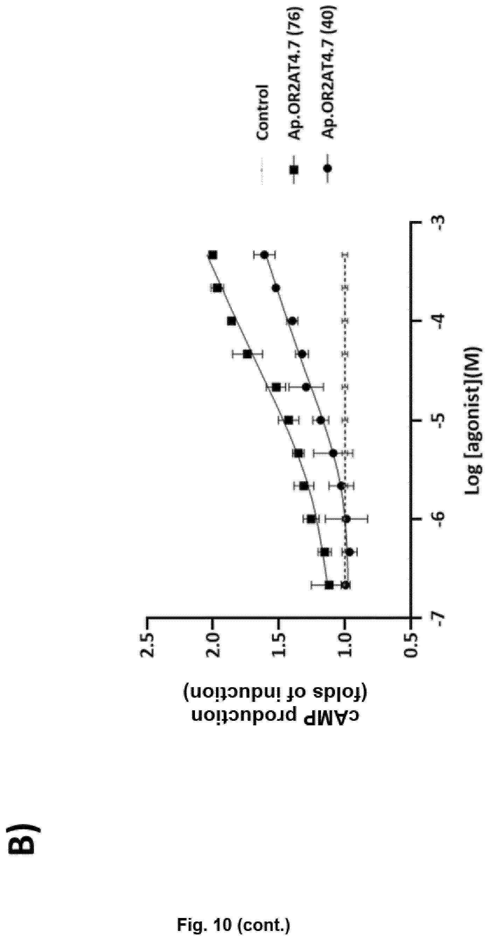

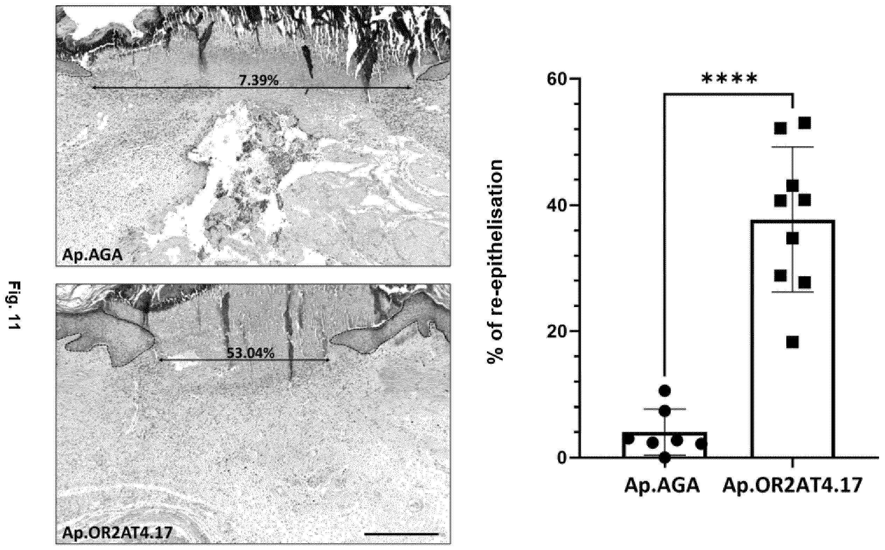

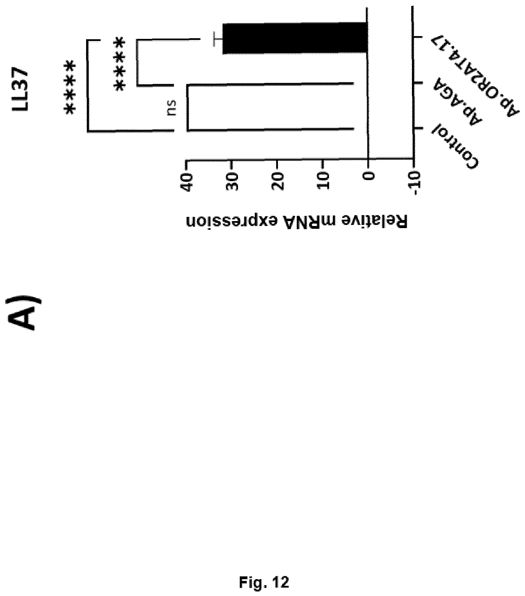

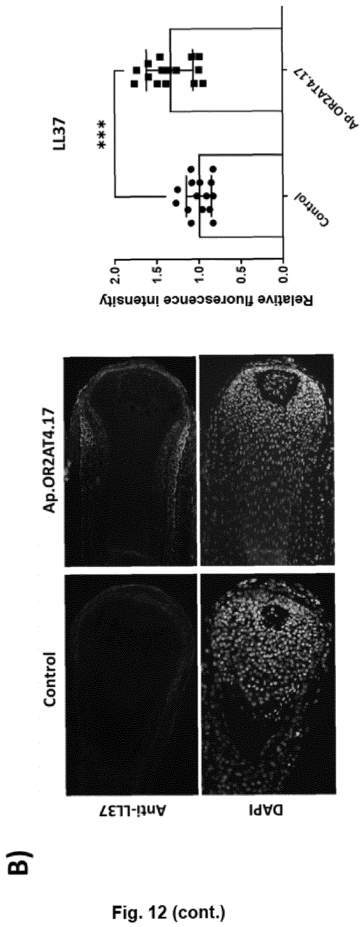

The aptamers of the present invention were obtained by means of the Cell-SELEX (Systematic Evolution of Ligands by Exponential Enrichment) method. After their generation, their capacity to bind to the OR2AT4 receptor, as well as the capacity to produce cAMP mediated by the activation of said receptor (Figure 3 and Figure 10), were then evaluated. Furthermore, the aptamers of the invention exhibited antiproliferative effects against cancer cells (Figure 4), the capacity to induce keratinocyte proliferation and migration (Figure 7 and Figure 8), as well as the prolongation of the anagen phase and an increased elongation of the hair shaft in human hair follicles (Figure 9), the capacity to promote wound healing in a skin-humanised mouse model (Figure 11), and the capacity to induce the expression of antimicrobial peptides in keratinocytes and hair follicle (Figure 12). Therefore, the specific binding to the OR2AT4 receptor and its activation mediated by the aptamers of the invention forms a new route for applications in the field of biomedicine and cosmetics.

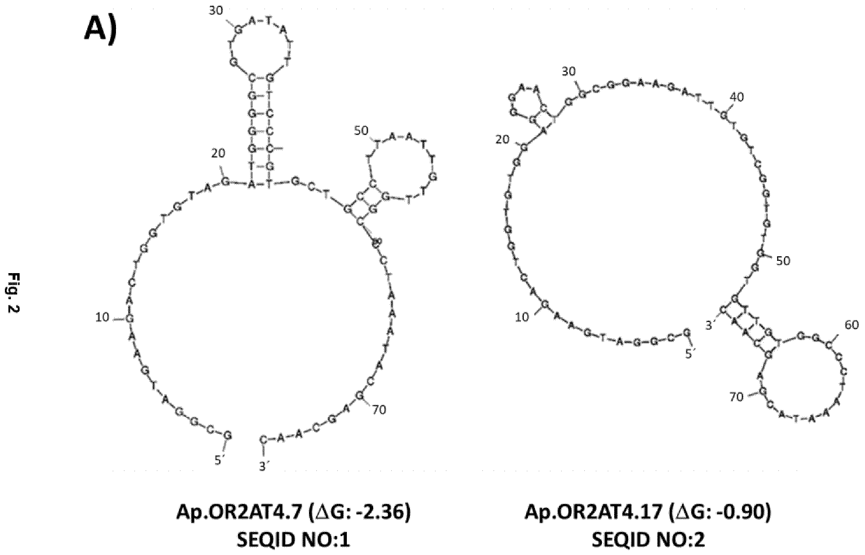

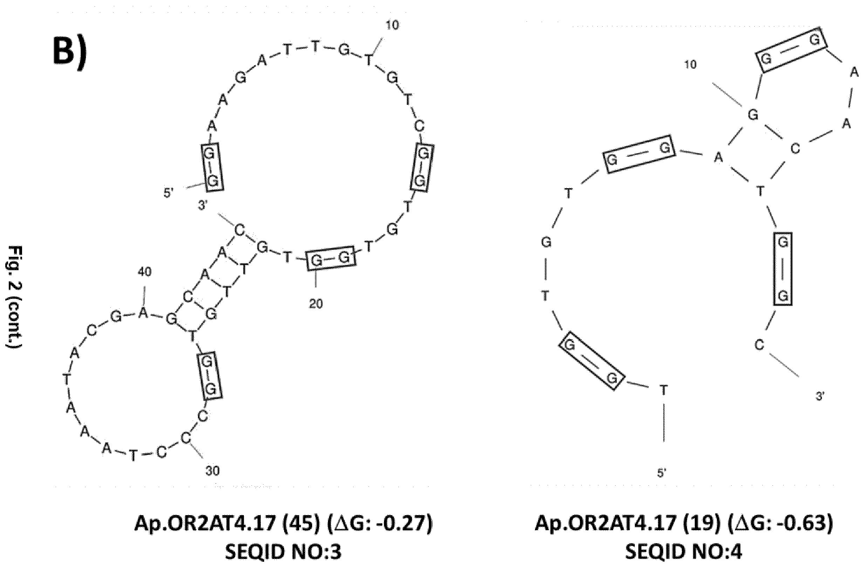

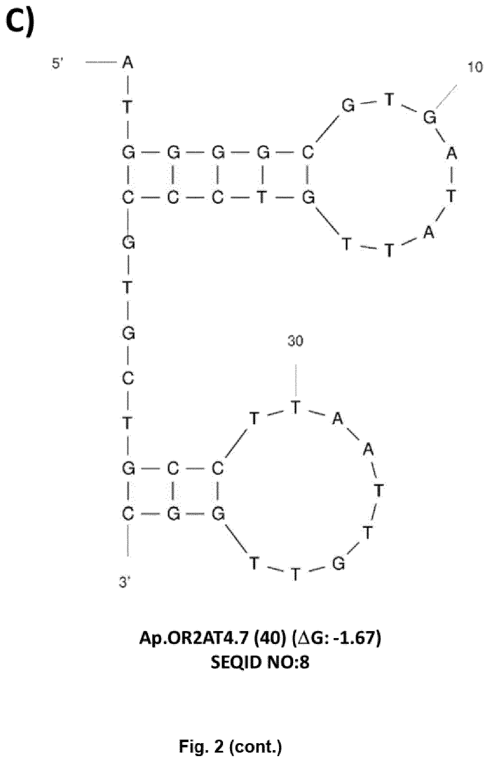

Aptamers are single-stranded DNA or RNA molecules which, based on their three-dimensional structure (Figure 2), are capable of recognising with high affinity and specificity the target molecule against which the aptamers have been generated. They exhibit recognition properties similar to those of antibodies, although the unique characteristics of their composition make them advantageous over antibodies as biotechnological platforms for both diagnosis and therapeutic applications. The use of antibodies in clinical trials exhibits certain limitations, such as those relating to the production process that generally involves the use of animals or cells, as well as the variability between different batches. Aptamers can be generated in a reproducible manner by using conventional solid-phase synthetic methods at a low cost, and can furthermore be modified to increase their stability, as well as to add functional groups so as to implement their applicability. Furthermore, aptamers exhibit low or no immunogenicity, so they constitute very interesting alternative therapeutic tools with respect to the use of antibodies.

Therefore, in one aspect, the present invention relates to a nucleic acid aptamer or a fragment thereof, preferably a deoxyribonucleic acid aptamer, capable of binding specifically to the OR2AT4 receptor and activating said OR2AT4 receptor, hereinafter "aptamer of the invention" (referring both to the aptamer and to the fragment thereof), wherein said aptamer comprises a nucleotide sequence with a sequence identity of at least 80% with the nucleotide sequence SEQ ID NO: 1, SEQ ID NO: 2, SEQ ID NO: 3, SEQ ID NO: 4, or SEQ ID NO: 8.
SEQ ID NO: 1, nucleotide sequence of the aptamer Ap.OR2AT4.7, also referred to in the present invention as Ap.OR2AT4.7-76: 
GCGGATGAAGACTGGTGTAGATGGGGCGTGATATTGTCCCGTGCTGCCTTAATTG
TTGGCCCTAAATACGAGCAAC
SEQ ID NO: 2, nucleotide sequence of the aptamer Ap.OR2AT4.17, also referred to in the present invention as Ap.OR2AT4.17-76: 
GCGGATGAAGACTGGTGTGGAGGGAACTGGCGGAAGATTGTGTCGGTGTGGTGT
TGTGGCCCTAAATACGAGCAAC
SEQ ID NO: 3, nucleotide sequence of the aptamer Ap.OR2AT4.17-45: GGAAGATTGTGTCGGTGTGGTGTTGTGGCCCTAAATACGAGCAAC
SEQ ID NO: 4, nucleotide sequence of the aptamer Ap.OR2AT4.17-19: TGGTGTGGAGGGAACTGGC
SEQ ID NO: 8, nucleotide sequence of the aptamer Ap.OR2AT4.7-40: ATGGGGCGTGATATTGTCCCGTGCTGCCTTAATTGTTGGC

The term "aptamer(s)", in the context of the present invention, refers to single-stranded nucleic acid, preferably deoxyribonucleic acid, chains that adopt a specific tertiary structure which allows them to bind to molecular targets with high specificity and affinity, comparable to that of monoclonal antibodies, through interactions other than classic Watson-Crick base pairing interactions, such as electrostatic and ionic interactions.

The term "fragment" in relation to the aptamer of the invention, also called part or partial sequence of the aptamer of the invention, refers to an aptamer fragment in which at least one or more nucleotides have been eliminated from the 5' end and/or the 3' end, and in which it conserves the functionality described above (capacity to bind specifically to the OR2AT4 receptor and to activate said OR2AT4 receptor). In preferred embodiments, the aptamer fragment comprises no more than 15, 20, 25, 30, 35, 40, 45, 50, 55, 60, 65, or 70 nucleotides in length; or preferably the aptamer fragment comprises between 10 and 60 nucleotides in length, more preferably between 10 and 50 nucleotides, between 10 and 40 nucleotides, between 10 and 30 nucleotides, or between 15 and 30 nucleotides in length.

The term "nucleic acid" in the context of the present invention, refers to any type of nucleic acid, such as DNA and RNA, and to variants thereof, such as peptide nucleic acid (PNA), locked nucleic acid (LNA), as well as combinations thereof, modifications thereof, which include modified nucleotides, etc. The terms "nucleic acid" and "oligonucleotide", and "polynucleotide" are used interchangeably in the context of the present invention. Nucleic acids can be purified from natural sources, produced using recombinant expression systems and, optionally, purified, chemically synthesised, etc., where appropriate, for example, in the case of chemically synthesised molecules, nucleic acids may comprise nucleoside analogues such as analogues having chemically modified bases or sugars, backbone modifications, etc. A nucleic acid sequence is depicted in the 5' -> 3' direction unless otherwise indicated.

In the present invention, the term "OR2AT4" refers to the OR2AT4 (olfactory receptor family 2 subfamily AT member 4) cell receptor located on the cell surface of different tissues including, among them, olfactory sensory cells, but it is also expressed in dendritic cells, melanocytes, and keratinocytes. This receptor is known by another name which includes, without being limited to, OR11-265.

In humans, the OR2AT4 receptor is encoded by the *OR2AT4* gene (NCBI gene ID: 341152; NC_000011.10 Reference GRCh38.p14 Primary Assembly; Region 75081753..75096890). Transcription of the *OR2AT4* gene gives rise to the NCBI transcript, NM_001005285.2 the translation of which gives rise to the receptor with the sequence NP_001005285.1 (SEQ ID NO: 9).

Amino acid sequence of the OR2AT4 receptor, SEQ ID NO: 9:

MDATACNESVDGSPVFYLLGIPSLPETFFLPVFFIFLLFYLLILMGNALILVAVVAEPSLH
KPMYFFLINLSTLDILFTTTTVPKMLSLFLLGDRFLSFSSCLLQMYLFQSFTCSEAFILVV
MAYDRYVAICHPLHYPVLMNPQTNATLAASAWLTALLLPIPAVVRTSQMAYNSIAYIYH
CFCDHLAVVQASCSDTTPQTLMGFCIAMVVSFLPLLLVLLSYVHILASVLRISSLEGRAK
AFSTCSSHLLVVGTYYSSIAIAYVAYRADLPLDFHIMGNVVYAILTPILNPLIYTLRNRDV
KAAITKIMSQDPGCDRSI

In a particular embodiment, the OR2AT4 receptor is a receptor in a mouse, rat, rabbit, pig, cat, dog, horse, or non-human primate. In another particular embodiment of the aptamer of the invention, the OR2AT4 receptor is the human OR2AT4 receptor.

In the present invention, the OR2AT4 receptor preferably refers to the receptor of sequence NP_001005285.1 (NCBI).

As mentioned earlier, the aptamer of the invention is capable of binding specifically to the OR2AT4 receptor, in other words, it is capable of specifically recognising and binding to said receptor in order to activate it.

In the present invention, "specific binding" or "specific binding to the OR2AT4 receptor" is understood as the non-covalent physical association between two molecules, the aptamer of the invention and the OR2AT4 receptor. The binding between the aptamer of the invention and the OR2AT4 receptor is considered specific if the binding strength between both is at least 10 times at least 15 times, at least 20 times, at least 25 times, at least 50 times, at least 75 times, or at least 100 times greater than the binding strength between the aptamer of the invention and an irrelevant molecule. The binding between the aptamer of the invention and the OR2AT4 receptor is also considered specific if the disassociation equilibrium constant Kd is 10⁻³ M or lower, 10⁻⁴ M or lower, 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower under the conditions used, for example, under physiological conditions, cell culture conditions, or conditions that allow cell survival.

The capacity of the aptamer of the invention to bind specifically to the OR2AT4 receptor can be determined by means of a variety of assays available in the art. Preferably, the capacity of the aptamer of the invention to bind specifically to the OR2AT4 receptor is determined by means of *in vitro* binding assays, such as enzyme-linked oligonucleotide assay (ELONA), the enzyme-linked aptamer sorbent assay (ELASA), precipitation, and quantitative PCR (qPCR), or by fluorescence techniques such as aptahistochemistry, aptacytochemistry, fluorescence microscopy, or flow cytometry. Likewise, both the capacity to bind specifically to the OR2AT4 receptor and the affinity of the aptamer for the OR2AT4 receptor can be determined by means of techniques widely known to the person skilled in the art, such as gel mobility shift assay, surface plasmon resonance (SPR), kinetic capillary electrophoresis, and fluorescence binding assay. Briefly, fluorescence binding assay consists of the incubation of OR2AT4 receptor-coated magnetic beads with different concentrations (for example, from 0 to 100 nM) of the aptamer of the invention which is labelled (for example, with carboxyfluorescein, FAM), and the subsequent elution and detection of bound aptamers, the dissociation constants (Kd) are calculated by non-linear fit analysis.

As explained above at the start of this description, the aptamer of the invention is a OR2AT4 receptor agonist, in other words, the aptamer of the invention acts as an activator of said receptor. In the present invention, "activation of the OR2AT4 receptor" is understood as the presence of, or the increase in, the signal mediated by the OR2AT4 receptor with respect to its inactivated state. The activity of a receptor is considered to be increased by an activator or agonist when its activity or the signal mediated by the receptor is greater than the activity or signal of the receptor when it is not bound to any ligand. Increase in activity can be at least 1%, at least 5%, at least 10%, at least 20%, at least 30%, at least 40%, at least 50%, at least 60%, at least 70%, at least 80%, at least 90%, or at least 100% greater than the activity of the receptor when it is not bound to any ligand. The capacity of an aptamer of the invention to activate the OR2AT4 receptor can be determined by means of a large variety of assays available in the state of the art. Preferably, the capacity of the aptamer of the invention to activate an OR2AT4 is determined by means of *in vitro* assays with cells expressing recombinant OR2AT4 and a reporter gene the expression of which is associated with the activation of said recombinant receptors. The person skilled in the art will recognise that there are several variants of this method, depending on the cell and the recombinant gene used. An example of this assay is described in the examples of the present invention (Example 3).

The aptamer of the invention is capable of binding specifically to the OR2AT4 receptor and activating said receptor. The aptamer of the invention comprises a nucleotide sequence having a sequence identity of at least 80% with a nucleotide sequence selected from the group consisting of the sequences SEQ ID NO: 1, SEQ ID NO: 2, SEQ ID NO: 3, SEQ ID NO: 4, and SEQ ID NO:8.

In the present invention, "identity" or "sequence identity" is understood as the degree of similarity between two nucleotide or amino acid sequences obtained by means of aligning the two sequences. Depending on the number of common residues between the aligned sequences, a degree of identity expressed as a percentage will be obtained. The degree of identity between two nucleotide/amino acid sequences can be determined by conventional methods, for example, by means of standard sequence alignment algorithms known in the state of the art such as, for example, BLAST [Altschul S.F. et al. Basic local alignment search tool. J Mol Biol. 1990 Oct 5; 215(3):403-10]. The BLAST programs, for example, BLASTN, BLASTX, and TBLASTX, BLASTP and TBLASTN, are in the public domain on the website of The National Center for Biotechnology Information (NCBI).

The person skilled in the art understands that mutations in the nucleotide sequence of the aptamers that give rise to nucleotide substitutions in positions not critical for the functionality of the aptamer, and are evolutionarily neutral mutations that do not affect their overall structure or functionality, giving rise to variants functionally equivalent to the original nucleotide sequence. Said variants fall within the scope of the present invention. Therefore, all those nucleotide sequences having at least 80% of identity with nucleotide sequences 1, SEQ ID NO: 2, SEQ ID NO: 3, SEQ ID NO: 4, or SEQ ID NO: 8, and furthermore having the same function as said sequences, in other words, capable of binding specifically to the OR2AT4 receptor and activating said OR2AT4 receptor, are functionally equivalent variants which are also contemplated within the scope of protection of the present invention.

In the present invention "functionally equivalent variant" refers to aptamers with sequences substantially similar to the sequences SEQ ID NO: 1, SEQ ID NO: 2, SEQ ID NO:3, SEQ ID NO: 4, or SEQ ID NO: 8 which maintain their capacity to bind specifically to and activate the OR2AT4 receptor. A functionally equivalent variant of the aptamer of the invention can be a nucleic acid sequence derived from the sequence SEQ ID NO: 1, SEQ ID NO: 2, SEQ ID NO:3, or SEQ ID NO: 4, or SEQ ID NO: 8 comprising the addition, substitution, or modification of one or more nucleotides. By way of illustration, functionally equivalent variants of the aptamer of the invention include sequences comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 5' end of the sequence SEQ ID NO: 1, SEQ ID NO: 2, SEQ ID NO: 3, or SEQ ID NO: 4 and/or comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 3' end of the sequence SEQ ID NO: 1, SEQ ID NO: 2, SEQ ID NO: 3, SEQ ID NO: 4, or SEQ ID NO: 8 and maintaining the capacity to bind specifically to and activate the OR2AT4 receptor of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the capacity thereof to bind specifically to and activate the OR2AT4 receptor.

The present invention also includes aptamers comprising nucleotide sequences with a sequence identity of at least 85%, at least 90%, at least 91%, at least 92%, at least 93%, at least 94%, at least 95%, at least 96%, at least 97%, at least 98%, or at least 99% with sequences SEQ ID NO: 1, SEQ ID NO: 2, SEQ ID NO: 3, SEQ ID NO: 4, or SEQ ID NO: 8 and maintaining the capacity to bind specifically to and activate the OR2AT4 receptor of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the capacity thereof to bind specifically to and activate the OR2AT4 receptor.

Therefore, in a particular embodiment of the invention, the aptamer comprises a nucleotide sequence having a sequence identity of at least 80, 85, 90, 91, 92, 93, 94, 95, 96, 97, 98, or 99 % with the sequence SEQ ID NO: 1, SEQ ID NO: 2, SEQ ID NO:3, SEQ ID NO: 4, or SEQ ID NO: 8. In a more particular embodiment, the aptamer comprises a nucleotide sequence having a sequence identity of 100% with the sequence SEQ ID NO: 1, SEQ ID NO: 2, SEQ ID NO: 3, SEQ ID NO: 4, or SEQ ID NO: 8. In another even more particular embodiment, the aptamer of the invention consists of the sequence SEQ ID NO: 1, SEQ ID NO: 2, SEQ ID NO: 3, SEQ ID NO: 4, or SEQ ID NO: 8.

The aptamers of the present invention are nucleic acid aptamers, which can be DNA or RNA. In a particular embodiment, the aptamer of the invention is a DNA aptamer. Nevertheless, in the present invention it is also contemplated that the aptamer can be formed by nucleic acid variants and analogues and combinations thereof. Examples of nucleic acid variants and analogues and combinations thereof include, but are not limited to, modified nucleic acid backbones, substitution bonds, modified nucleotides, and ribose or deoxyribose analogues, modified nucleotides, etc. with the capacity to bind specifically to and activate the OR2AT4 receptor of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least the 100% of the capacity to bind specifically to and activate the OR2AT4 receptor of the aptamer comprising the sequence SEQ ID NO: 1, SEQ ID NO: 2, SEQ ID NO: 3, SEQ ID NO: 4, or SEQ ID NO: 8. Non-limiting examples of nucleic acid variants and analogues include, but are not limited to, APN, ANB and ATN.

The term "nucleic acid variant" or "nucleic acid analogue", in the context of the present invention, refers to nucleic acid variants and analogues which include, but are not limited to, modified nucleic acid backbones, substitution bonds, modified nucleotides, and ribose or deoxyribose analogues. For example, nucleic acid variants according to the present invention may comprise structures with synthetic backbones analogous to the typical phosphodiester backbone. These include, but are not limited to, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs), methylphosphonate bonds, or alternating methylphosphonate and phosphodiester and benzylphosphonate.

Nucleic acid variants may also contain one or more "substitution" bonds, as generally understood in the art. Some of these substitution bonds are non-polar and contribute to providing the aptamer with a capacity to diffuse across membranes. These "substitution" bonds are defined herein as conventional alternative bonds such as phosphorothioate or phosphoramidate, and are synthesised as described in commonly available literature. Alternative binding groups include, in a non-limiting manner, embodiments in which a moiety of formula P(O)S, ("lioalo"), P(S)S ("dilioalo"), P(O)NR' P(O)R', P(O)OR', CO, or CONR', wherein R' is H (or a salt) or an alkyl group with 1-12 C atoms and R6 is an alkyl group with 1-9 C atoms, which bind to adjacent nucleotides through -S-or -O-. The present invention also contemplates the use of substitution bonds which include non-phosphorous-based internucleotide bonds such as 3'-thioformacetal, (-S-CH2-O-), formacetal (-O-CH2-O-), and 3'amine internucleotide bonds (-NH-CH2-CH:d). One or more substitution bonds can be used in the aptamers of the invention in order to further facilitate binding to the OR2AT4 receptor, or to increase the stability of the aptamers against nucleases, as well as to confer permeation capacity. Not all bonds within the same aptamer have to be identical and therefore, the present invention contemplates aptamers with all identical bonds, as well as aptamers with a variation in the composition of their bonds.

Likewise, nucleic acid variants according to the present invention may also contain analogous forms of ribose or deoxyribose which are well known in the art, including, but are not limited, to 2' substituted sugars such as 2'-O-methyl ribose, 2'-fluororibose or 2'-azido-ribose, carbocyclic analogues of sugars, α-anomeric sugars, epimeric sugars such as arabinose, xyloses, or lyxoses pyranose sugars, furanose sugars, and sedoheptuloses. Nucleic acids may also contain threose nucleic acid (TNA), also referred to as alpha-threofuranosyl oligonucleotides). In particular, substitution at the 2' position of the furanose residue is particularly important with respect to improving nuclease stability.

The term "nucleotide", in the context of the present invention, refers to the monomers making up nucleic acids. Nucleotides are formed by a pentose, a nitrogenous base, and a phosphate group, and are bound by phosphodiester bonds. Nucleotides that are part of DNA and RNA differ in the pentose, which is deoxyribose and ribose, respectively. In turn, nitrogenous bases are divided into purine nitrogenous bases, which are adenine (A) and guanine (G), and pyrimidine nitrogenous bases, which are thymine (T), cytosine (C), and uracil (U). Thymine can be found only in DNA, while uracil can be found only in RNA. The present invention contemplates the use of modified nucleotides in the aptamer of the invention. The term "modified nucleotide", in the context of the present invention, refers to known analogues of natural nucleotides, with similar or improved binding properties. Analogous forms of purines and pyrimidines are well known in the art, and include, but are not limited to, aziridinylcytosine, 4-acetylcytosine, 5-fluorouracil, 5-bromouracil, 5-carboxymethylaminomethyl-2-thiouracil, 5-carboxymethylaminomethyluracil, inosine, N₆-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N₆₋methyladenine, 7-methylguanine, 5-methylaminomethyluracil, 5-methoxyaminomethyl-2-thiouracil, beta-D-manosylqueosine, 5-methoxyuracil, 2-methylthio-N-6-isopentenyladenine, uracyl-5-oxyacetic acid methyl ester, pseudouracil, queosine, 2-thiocytosine, 5-methyl-2-thiouracil, 2-thiouracil, 4-thiouracil, 5-methyluracil, uracil-5-oxyacetic acid, and 2,6-diaminopurine. In addition to the preceding modified nucleotides, nucleotide residues lacking a purine or a pyrimidine can also be included in the present invention.

In addition to the preceding variants, nucleic acid variants comprised in the invention also include PNA, LNA, and 5'-5' or 3'-3' chains. The term "peptidonucleic acid" or "PNA", in the context of the present invention, refers to an oligonucleotide the backbone of which is composed of repeating units of N-(2-aminoethyl)glycine bound by peptide bonds, wherein the different nitrogenous bases are bound to the main chain by a methylene bond (-CHT) and a carbonyl group (-(C=O)). The term "locked nucleic acid" or "LNA", in the context of the present invention, refers to a modified RNA nucleotide the ribose moiety of which is modified with an additional bond that connects oxygen at 2' to carbon at 4', locking ribose in the 3'-endo conformation. The term "5'-5' chain" or "3'-3' chain", in the context of the present invention, refers to oligonucleotides in which the nucleotide at the 3' or 5' end, respectively, is inverted.

The aptamer of the invention comprises a variable size of nucleotides. In a particular embodiment, it comprises a size between 19 and 200 nucleotides, preferably between 19 and 150 nucleotides, more preferably between 19 and 100 nucleotides.

Production of the aptamer of the invention can be carried out following conventional methods in the art. Non-limiting examples of aptamer production techniques include enzymatic techniques, such as transcription, recombinant expression systems, and standard solid phase (or solution phase) chemical synthesis, that are commercially available. Where appropriate, for example, in the event that the aptamer of the invention comprises nucleic acid variants such as those described above, nucleotide analogues such as analogues having chemically modified bases or sugars, backbone modifications, etc., the aptamer of the invention will be produced by means of chemical synthesis. Alternatively, recombinant expression will be the preferred technique for the production of aptamers when they are 200 nucleotides or longer in length. Aptamers produced by any of the preceding techniques can, optionally, be purified by methods well known in the art.

As will be appreciated by the person skilled in the art, the features of small size, stability, and easy production of the aptamer of the invention, mean that the aptamer can be presented bound to a second molecule. This is particularly advantageous when the second molecule is a functional group. The binding of the aptamer of the invention and a functional group results in a complex which exhibits the combination of functions of both, in other words, a complex capable of binding specifically to and activating the OR2AT4 receptor and with the activity associated with the functional group.

Therefore, in another aspect, the present invention relates to a complex, hereinafter referred to as the "complex of the invention", comprising the aptamer of the invention and a functional group.

The term "aptamer" has been described in detail in the previous inventive aspect (aptamer of the invention) and its definitions and particularities also apply to the complex of the invention.

The term "functional group", in the context of the present invention, refers to molecules capable of performing at least one function. Said function includes, but are not limited to, the capacity to specifically bind to the OR2AT4 receptor, and to activate it, the capacity to be detectable, both directly and indirectly, the capacity to induce cell death, etc. As will be understood by the person skilled in the art, a functional group may have one or more functions associated therewith. Non-limiting examples of functional groups include detectable reagents, drugs, and nanoparticles. These functional groups can act as imaging agents, drugs, etc. Therefore, in a particular embodiment, the functional group of the complex of the invention is a detectable reagent, a drug, or a nanoparticle.

The term "detectable reagent", in the context of the present invention, refers to reagents capable of being detected directly or indirectly. Examples of detectable reagents indicated for the present invention include, but are not limited to, radionuclides, fluorophores, proteins, and haptens.

In a preferred embodiment, the detectable reagent is a radionuclide. To that end, appropriate radionuclides are useful in diagnostic imaging techniques, such as radio immunodiagnostic techniques and positron emission tomography (PET). Non-limiting examples of radionuclides include gamma emitting isotopes, such as 9^{9m}Tc, ¹²³I, and ¹¹¹In, which can be used in radio scintigraphy using gamma cameras or single photon emission computerised tomography, as well as positron emitters, such as ¹⁸F, ⁶⁴Cu, ⁶⁸Ga, ⁸⁶Y, ¹²⁴I, ²¹³Bi and ²¹¹At, which can be used in PET, o beta emitters, such as ¹³¹I, ⁹⁰Y, ^{99m}Tc, ¹⁷⁷Lu and ⁶⁷Cu. The person skilled in the art will appreciate that radionuclides can also be used for therapeutic purposes.

In another preferred embodiment, the detectable reagent is a fluorophore. The term "fluorophore", in the context of the present invention, refers to a fluorescent chemical compound that can emit light after being excited by light having a different wavelength. Suitable fluorophores in the present invention include, but are not limited to, Cy3, Cy2, Cy5, the Alexa Fluor^{®} family of fluorescent markers (Molecular Probes,. Inc.), carboxyfluorescein (FAM), and fluorescein isothiocyanate (FITC).

In another preferred embodiment, the detectable reagent is a protein. The term "protein" in the context of the present invention, refers to macromolecules consisting of one or more amino acid chains. Proteins are responsible for carrying out a diverse group of cellular functions based on their ability to bind other molecules specifically. Proteins can bind to other proteins as well as to small substrate molecules. Non-limiting examples of proteins suitable for the purposes of the present invention include, but are not limited to, enzymes, fluorescent proteins, luminescent proteins, and antigens.

In an even more preferred embodiment, the protein is an enzyme. The term "enzyme" in the context of the present invention, refers to a protein that functions as a highly selective catalyst, accelerating both the speed and specificity of the metabolic reaction for which it is specific. Non-limiting examples of enzymes suitable for the invention include, but are not limited to, horseradish peroxidase (HRP) and alkaline phosphatase. As will be appreciated by the person skilled in the art, enzymes suitable for use in the present invention are indirectly detectable as a result of their capacity to catalyse the modification of a substrate into a compound detectable by colorimetry, chemiluminescence, or fluorimetry. Examples of suitable substrates include, but are not limited to, p-nitrophenyl phosphate (PNPP), 2,2'-azinobis [3-ethylbenzothiazoline-6-sulphonic acid] (ABTS), o-phenylenediamine (OPD), and 3,3',5,5'-tetramethylbenzidine (TMB).

Bioluminescent proteins or photoproteins are a particular case of oxidative enzymes which are capable of carrying out a chemical reaction of their specific prosthetic groups, resulting in light emission without the need for prior excitation. Non-limiting examples of bioluminescent proteins include firefly luciferase, *Renilla* luciferase, and the aequorin

In another even more preferred embodiment, the protein is a fluorescent protein. The term "fluorescent protein", in the context of the present invention, refers to a protein capable of emitting light when excited at a wavelength suitable for excitation. Non-limiting examples of fluorescent proteins that can be used in the complex of the invention include, but are not limited to, GFP, GFPuv, BFP, CFP, YFP, EBFP2, mCerulean, mCerulean3, mVenus, mTurquoise, T-Sapphire, citrine, amFP486, zFP506, zFP538, drFP, DsRed, mCherry, dTomato, mTFP1, TagRFP-T, mKO2, mRuby, mKate, mAmetrine, REACh, R-phycoerythrin (R-PE), and allophycocyanin (APC).

In another even more preferred embodiment, the protein is a luminescent protein. The term "luminescent protein", in the context of the present invention, refers to a protein capable of emitting light when excited at a wavelength suitable for excitation.

In another even more preferred embodiment, the protein is an antigen. The term "antigen", in the context of the present invention, refers to a molecule which induces an immune response in the body. Therefore, an antigen can be used to generate an antibody that recognises it and binds specifically to it. Non-limiting examples of antigens include, among others, tumor antigens, such as carcinoembryonic antigen (CEA), HER2, prostate specific antigen (PSA) and plasminogen tissue activator and the recombinant variants thereof such as Activase^{®}, as well as bacterial antigens, allergens, etc. As will be appreciated by the person skilled in the art, the antigens suitable for use thereof in the present invention are indirectly detectable as a result of their capacity to be specifically recognised by an antibody.

In another preferred embodiment, the detectable reagent is a hapten. The term "hapten", in the context of the present invention, refers to a group of chemical compounds with a small molecular size (generally, less than 10,000 Da) that are antigenic but incapable of inducing a specific immune reaction by themselves. The chemical coupling of a hapten to a large immunogenic protein, called a carrier, generates an immunogenic hapten-carrier conjugate which is capable inducing a specific immune reaction. Non-limiting examples of vitamins include biotin (vitamin B7), digoxigenin, dinitrophenol (DNP), and nitro-iodophenol (NIP). In a more preferred embodiment, the vitamin is biotin. The term "biotin", in the context of the present invention, refers to a heat-stable, water- and alcohol-soluble vitamin, also called vitamin H and vitamin B7, characterised by binding specifically to avidin with the highest affinity reported to date of Kd = 10¹⁵ M. As will be appreciated by the person skilled in the art, biotin is indirectly detectable as a result of its capacity of being specifically recognised by avidin or variants thereof such as streptavidin and neutravidin.

In another particular embodiment, the functional group is a drug. The term "drug" in the context of the present invention, refers to a chemical substance used in the treatment, cure, or prevention of a disease, condition, or pathology characterised by a decrease or inhibition of the expression of the OR2AT4 receptor, and/or a decrease in the activation of the OR2AT4 receptor. It also refers to that chemical substance used in the treatment, cure, or prevention of a disease, condition, or pathology characterised by the unavailability of the endogenous ligands of the OR2AT4 receptor or, even with endogenous ligand availability, an extra supply of exogenous ligand could be beneficial. Therefore, these receptors have been shown to be involved in cancer development and progression. Furthermore, OR2AT4 is involved in the hair follicle growth cycle, therefore, it has characteristics that make it useful as a target in the treatment of conditions such as anagen effluvium, telogen effluvium, or alopecia, particularly androgenetic alopecia. Therefore, the diseases or conditions that can be treated with OR2AT4 aptamers are all those included in the following categories: cancer, alopecia, effluvium (telogen or anagen), wounds, and dermatoses (preferably dermatoses caused by dysbiosis).

The present invention contemplates that the drug is a nucleic acid. Therefore, in a preferred embodiment the drug is a nucleic acid. Nucleic acids suitable as drugs in the context of the complex of the invention include antisense RNA, antisense DNA, and small interfering RNAs.

The present invention contemplates that the drug is a peptide. Therefore, in a preferred embodiment the drug is a peptide. The term "peptide", in the context of the present invention, refers to a short chain of amino acids linked by peptide bonds. The peptide will comprise at least 2 amino acids, at least 3 amino acids, at least 4 amino acids, at least 5 amino acids, at least 10 amino acids, at least 15 amino acids, at least 20 amino acids, at least 30 acidic amino acids, at least 40 amino acids, at least 50 amino acids, at least 60 amino acids, or at least 70 amino acids. Suitable for the purposes of this invention are, among others, peptides capable of binding to a target and inducing or inhibiting cell signalling. The term "target binding peptide", in the context of the present invention, refers to a peptide comprising a target binding region. The term "signaling peptide", in the context of the present invention, refers to a peptide capable of causing cell signalling, such as cell receptor agonist peptides. Suitable amino acid sequences for the binding of target molecules include molecular recognition consensus sequences well known in the art.

In another particular embodiment, the functional group is a nanoparticle. As it is used herein, refers to colloidal systems of the spherical, cylindrical, polyhedral, etc., or similar shapes, having a size of less than or equal to 1 µm, which can be found individually or forming organised structures (dimer, trimers, etc.), dispersed in a fluid (aqueous solution). In a particular embodiment, the nanoparticles suitable for putting the invention into practice have a size of less than 1 µm, generally comprised between 1 and 99 nanometers (nm), typically between 5 and 500 nm, preferably between about 10 and 150 nm. In a particular embodiment, the nanoparticles have a mean diameter of between 2 and 50 nm, preferably, between 5 and 20 nm. The mean particle diameter is the maximum mean particle dimension, where it is understood that the nanoparticles do not necessarily have to be spherical. Nanoparticles suitable for use in the present invention include polymeric nanoparticles, lipid nanoparticles, and metallic nanoparticles.

Polymeric nanoparticles are formed by a polymer matrix that is anchored to the aptamer of the invention. Examples of biocompatible polymers that can be used in the context of the present invention include, but are not limited to, polyethylenes, polycarbonates, and polyanhydrides.

Alternatively, the nanoparticles that act as functional groups can be lipid nanoparticles such as a liposome or a micelle. The formation of micelles and liposomes from, for example, vesicle-forming lipids, is widely known in the state of the art,

Likewise, the nanoparticle can be a metallic nanoparticle. The term "metallic nanoparticle" refers to nanoparticles comprising a metal and comprises the property known as surface plasmon phenomenon. The surface plasmon of a metal can be measured by means of any spectroscopic technique of the state of the art, such as surface plasmon resonance spectroscopy.

In a particular embodiment, the nanoparticle is a silica mesoporous magnetic nanoparticle.

Nanoparticles can be functionalised by means of adding a coating to the surface thereof. For biological applications, the coating surface should be polar to provide high water solubility and prevent nanoparticle aggregation.

The aptamer of the invention can be bound to the nanoparticle through covalent bonds, preferably, on the surface of the nanoparticle.

The binding between an aptamer of the invention and a functional group to generate the complex of the invention can be carried out by means of conjugation techniques widely known to the person skilled in the art. The result is a covalent bond between the aptamer of the invention and the functional group. The conjugation may involve the binding of primary amines at the 3' or 5' ends of the aptamer of the invention to the functional group during the chemical synthesis of the aptamer. Alternatively, the conjugation can be carried out by means of conventional cross-linking reactions, which have the advantage of the much higher chemical reactivity of primary alkyl amine tags compared to the aryl amines of the nucleotides themselves. Conjugation methods are well known in the art and are based on the use of cross-linking reagents. Cross-linking reagents contain at least two reagent groups that target groups such as primary amines, sulfhydryls, aldehydes, carboxyls, hydroxyls, azides, and so on, in the molecule to be conjugated. Cross-linking agents differ in chemical specificity, the length of the spacer arm, the composition of the spacer arm, cleavage spacer arm, and structure. For example, the conjugation of complexes according to the invention can be carried out directly or through a linking moiety, through one or more non-functional groups in the aptamer and/or the functional group, such as amine groups, carboxyl, phenyl, thiol, or hydroxyl. More selective bonds can be achieved by means of using a heterobifunctional linker. Conventional linkers can be used, such as diisiocyanates, diisothiocyanates, bis (hydroxysuccinimide)esters, carbodiimides, maleimide-hydroxysuccinimide esters, glutaraldehyde, and the like, hydrazines and hydrazides, such as butyric acid 4-(4-N-maleimidophenyl) hydrazide(MPBH).

Another approach consists of the marking of aptamers during PCR synthesis by means of using primers labelled, for example, with a fluorophore. To that end, there are various companies available to the person skilled in the art.

Additionally, in the particular embodiment in which the functional group is a radionuclide, the binding between an aptamer according to the invention and the radionuclide can be carried out by means of chemical coordination, wherein the aptamer atoms involved in the binding donate electrons to the radionuclide. Coordination reactions are widely known in the art and will depend on the radionuclide and reagent group involved in the aptamer.

According to the results included in this application, the aptamer of the invention is capable of activating the OR2AT4 receptor, but when the aptamer is combined with Sandalore, a significant increase in the activation of the OR2AT4 receptor occurs, preferably a synergistic effect occurs (Figure 3).

Therefore, another aspect of the present invention relates to a combination, hereinafter "combination of invention" comprising:
i) the aptamer or complex of the invention; and
ii) a compound of formula (I): 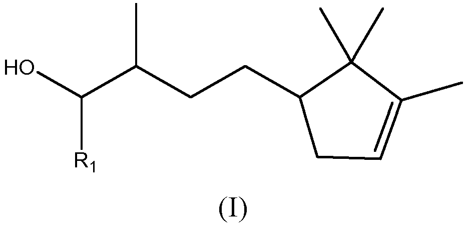
wherein R1 of formula (I) is a hydrogen (H) or a methyl group (-CH₃).

The compound of formula (I) wherein R₁ is a methyl is also referred to as Sandalore (CAS number: 65113-99-7, sandal pentanol).

The compound of formula (I) wherein R₁ is a hydrogen is also referred to as Brahmanol (CAS number: 72089-08-8, sandal cyclopentane).

In another preferred embodiment of the combination of the invention, R₁ in the compound of formula (I) is a methyl group.

In a preferred embodiment, the combination of the invention is comprised in a composition, preferably a cosmetic composition or a pharmaceutical composition, preferably formulated in liquid, gel, or foam form.

In another aspect, the present invention relates to a composition, hereinafter, "composition of the invention", comprising the aptamer of the invention, the complex of the invention, or the combination of the invention.

In a preferred embodiment, the composition of the invention is a pharmaceutical composition (if it is to be administered for therapeutic purposes) or a cosmetic composition (if it is to be administered for cosmetic purposes).

As it is used herein, the term "pharmaceutical composition" refers to a composition or mixture comprising an active agent (for example, the aptamer, complex, or combination of the invention), in which the active agent produces a physiological result or modulates a biological process when administered to a subject, for example, health benefits. The active agents are distinguished from other components of the administration compositions, as carriers, diluents, binders, colourants, etc.

The term "cosmetic composition" designates any substance or mixture intended to be placed in contact with the different external parts of the body of a subject (epidermis, hair system, nails, lips, and external genital organs) or with the teeth and the mucous membranes of the oral cavity for the main exclusive purpose of cleaning them, perfuming them, changing/improving their appearance, and/or correcting body odours, and/or protecting them or keeping them in good condition.

In a preferred embodiment, the composition of the invention further comprises an acceptable excipient and/or vehicle; preferably a pharmacologically or cosmetically acceptable excipient and/or vehicle.

Acceptable excipients or vehicles are widely known to the person skilled in the art and intended to include any solvent, dispersion medium, coatings, antibacterial and antifungal agents, isotonic and absorption retarding agents, and the like, compatible with pharmaceutical or cosmetic administration. Acceptable vehicles, excipients, or acceptable stabilizers are not toxic to the subject at the doses and concentrations used, and include, but are not limited to, buffers such as phosphate, citrate, and other organic acids; antioxidants including ascorbic acid and methionine; preservatives (such as octadecyldimethylbenzyl ammonium chloride, hexamethonium chloride, benzalkonium chloride, benzethonium chloride; phenol, butyl, or benzyl alcohol; alkyl parabens such as methyl or propyl paraben; catechol; resorcinol; cyclohexanol; 3-pentanol, and m-cresol); low molecular weight polypeptides (less than about 10 amino acids); proteins, such as serum albumin, gelatin, or immunoglobulins; hydrophilic polymers such as polyvinylpyrrolidone; amino acids such as glycine, glutamine asparagine, histidine, arginine, or lysine; monosaccharides, disaccharides, and other carbohydrates including glucose, mannose, or dextrins; chelating agents such as EDTA; sugars such as sucrose, mannitol, trehalose, or sorbitol; salt-forming counter ions such as sodium; metal complexes (for example, Zn-protein complexes); and/or nonionic surfactants such as TWEEN^{™}, PLURONICS^{™}, or polyethylene glycol (PEG).

The aptamers, the complex, or the combination of the invention may be in the composition of the invention in a therapeutically or cosmetically effective amount. The term "a therapeutically effective amount", in the context of the present invention, refers to the amount of the aptamer, complex, or combination of the invention that is required to achieve prevention, curing, delay, reduction in severity of, or improvement of one or more appreciable symptoms of the disease or pathological condition. The term "a cosmetically effective amount" refers to the amount of the aptamer, complex, or combination of the invention that is required to achieve a non-therapeutic cosmetic effect (for example, an amount that results in the preservation of skin integrity, improvement of skin appearance, or improvement of hair appearance).

In a preferred embodiment, the composition of the invention can be administered to a subject by means of any suitable route of administration, such as, for example, without being limited to, parenteral route, intramuscular route, subcutaneous route, oral route, topical route, ophthalmic route, inhalation route, or intranasal route.

In a preferred embodiment, the composition of the invention is in the form of an aqueous or oily solution, powder, cream, gel, emulsion, foam, tablets, pills, pills, aqueous or oily suspensions, or dispersible granules, emulsions, hard or soft capsules, or syrups or elixirs.

The aptamer of the invention is capable of recognising the OR2AT4 receptor, specifically binding thereto, and activating the signalling associated with said receptors, just as their natural ligands do. This capacity of the aptamer of the invention can then be used to detect the presence of the receptor by means of detecting the aptamer-receptor binding.

Therefore, in another aspect, the present invention relates to the *in vitro* use of the aptamer of the invention or complex of the invention to detect the OR2AT4 receptor, hereinafter "use for determination of the invention".

In a preferred embodiment of the use for determination of the invention, the functional group of the complex is selected from a detectable reagent and a nanoparticle, preferably a detectable reagent.

Therefore, the capacity of an aptamer according to the invention to specifically bind to the OR2AT4 receptor can be exploited for the indirect detection of said receptor.

For this purpose, the person skilled in the art will recognise that subsequent detection of said aptamer or complex is necessary. Aptamer detection techniques are widely known in the art and include, for example, the use of antibodies or probes specific for the aptamer. In this way, once the aptamer of the invention is bound to the OR2AT4 receptor, an antibody or probe specific for the aptamer, which in turn can be labelled with a detectable reagent or detected indirectly by means of a secondary antibody or probe, would be applied. The technique used for detecting the OR2AT4 receptor will then depend on the type of detectable reagent, being able to be techniques that are based, for example, on fluorimetry, colorimetry, or radioactivity.

The term "probe" or "hybridisation probe", in the context of the present invention, refers to a DNA or RNA fragment of variable length, generally between 10 and 1,000 bases in length, which is used to detect the presence of single-stranded nucleic acids (DNA or RNA) that are complementary to the sequence in the probe. The probe hybridises to the target single-stranded nucleic acid, the base sequence of which allows base pairing due to the complementarity between the probe and the target nucleic acid. To detect the hybridisation of the probe to its target sequence, the probe is labelled with a detectable reagent, such as a radionuclide, a fluorophore, or digoxygenin, among others.

Detection of the binding of the OR2AT4 receptor with the aptamer or complex of the invention can be carried out by means of *in vitro* binding assays, such as enzyme-linked oligonucleotide assay (ELONA), enzyme-linked aptamer sorbent assay (ELASA), precipitation, and quantitative PCR (qPCR), gel mobility shift assay, Western blotting, surface plasmon resonance (SPR), kinetic capillary electrophoresis, fluorescence binding assay, aptahistochemistry, aptacytochemistry, fluorescence microscopy, or flow cytometry.

In another particular embodiment of the uses for determination of the invention, detection of the OR2AT4 receptor is performed by means of a method selected from the group consisting of ELONA, aptacytochemistry, aptahistochemistry, and flow cytometry.

As described previously, both the aptamer of the invention and the complex of the invention are capable of specifically binding to and activating the OR2AT4 receptor.

Therefore, in another aspect, the present invention relates to the *in vitro* use of the aptamer of the invention, the complex of the invention, or the combination of the invention to activate the OR2AT4 receptor, hereinafter "use for activation of the invention".

In a manner similar to the *in vitro* uses described in preceding paragraphs, the present invention also relates to an *in vitro* method for detecting the OR2AT4 receptor in a biological sample isolated from a subject, and an *in vitro* method for activating the OR2AT4 receptor in a biological sample isolated from a subject.

Therefore, in another aspect the present invention relates to an *in vitro* method for detecting the OR2AT4 receptor in a biological sample isolated from a subject, hereinafter "method for detection of the invention" or "first *in vitro* method of the invention, which comprises the following steps:
a) contacting the isolated biological sample with the aptamer or complex of the invention,
b) separating the aptamer or complex not bound to the OR2AT4 receptor, and
c) detecting the presence of the aptamer or complex bound to the OR2AT4 receptor present in the isolated biological sample.

In the present invention, "sample" or "biological sample" is understood as the cell culture or biological material isolated from a subject. The biological sample may contain any biological material suitable for detecting the desired biomarker and may comprise cells and/or non-cellular material from the subject. The sample can be isolated from any suitable tissue or biological fluid such as, for example, blood, plasma, serum, urine, cerebrospinal fluid (CSF), heart, brain, or epithelial tissue.

The term "subject" or "individual" refers to a member of a mammalian species, and includes, but is not limited to domestic animals, and primates including humans; the subject is preferably a human, man or woman, of any age or race.

The aptamer or complex of the invention is applied to the sample in a suitable buffer to allow the binding of the aptamer or complex to OR2AT4 receptor molecules that may be present in the sample. Non-limiting examples of suitable buffers to allow the binding of the aptamer or complex of the invention to the OR2AT4 receptor molecules include, but are not limited to, PBS, TBS, phosphate buffer, and citrate buffer. Preferably, these buffers contain 1 mM MgCl₂. The amount of aptamer or complex of the invention necessary to detect the OR2AT4 receptor molecules present in the sample will depend on both the size of the sample and the amount of OR2AT4 receptor present therein, and can be easily determined by optimisation methods that are common in the art.

The aptamer or complex of the invention is incubated with the sample at a suitable temperature and for a time sufficient to allow the binding of the aptamer or complex to OR2AT4 receptor molecules that may be present in the sample. The temperature is, preferably, between 20°C and 37°C. As a guide, the aptamer will be incubated with the sample for at least 5 minutes, at least 10 minutes, at least 15 minutes, at least 20 minutes, at least 30 minutes, at least 60 minutes, at least 120 minutes or more.

Once the aptamer or complex of the invention has bound to the OR2AT4 receptor molecules that may be present in the sample, in step b) of the method for determination of the invention, the sample is washed to remove any aptamer or complex molecules that have not bound to the OR2AT4 receptor.

After washing, in step c) of the method for determination of the invention, the presence of the aptamer or complex of the invention bound to the OR2AT4 receptor present in the sample is detected.

Since the aptamer of the invention is not itself a detectable molecule, the detection step is an indirect detection step through a second detectable molecule that binds specifically to the aptamer. Detection of the aptamer bound to the OR2AT4 receptor can be carried out with virtually any known antibody or reagent that binds with high affinity to the aptamer of the invention. Nevertheless, the use of an antibody specific for the aptamer is preferable, for example, polyclonal serum, hybridoma supernatants, monoclonal or humanised antibodies and fragments thereof. Said antibody specific for the aptamer is suitably labelled with a detectable reagent. The term "detectable reagent" has been described in detail above and its definition and particularities are included herein by reference. Said reagent can be detected by fluorimetry or colorimetry using apparatus suitable for the type of reagents and the type of sample, which are known to the person skilled in the art. By way of example, the sample with the aptamer bound to the OR2AT4 receptor molecules present is incubated with an antibody specific for the aptamer is conjugated to an enzyme, under conditions similar to the incubation conditions with the aptamer, and OR2AT4-aptamer-antibody complexes are detected with the addition of a substrate that is converted by the enzyme to a detectable product, for example, by means of fluorimetry in a fluorescence microscope or by colorimetry in a spectrophotometer. Alternatively, detection can be performed in a similar manner by means of using an aptamer-specific probe suitably labelled with a detectable reagent. The person skilled in the art will recognise that the first *in vitro* method of the invention can be carried out as part of detection techniques such as ELONA, ELASA, precipitation and qPCR, gel mobility shift assay, Western blotting, surface plasmon resonance, kinetic capillary electrophoresis, fluorescence binding assay, aptahistochemistry, aptacytochemistry, fluorescence microscopy, or flow cytometry.

As will be appreciated by the person skilled in the art, the detectable reagents contemplated by the present invention can be divided into reagents that are directly detectable *per se,* such as radionuclides or fluorophores, and reagents that are indirectly detectable, such as proteins or haptens. In a particular embodiment, the detectable reagent is a radionuclide and detection is performed by detecting the radiation emitted by the radionuclide. Said radiation will depend on the type of radionuclide, being able to be an emission of α particles, β particles, or γ-type emission. To that end, suitable detection techniques for different radionuclides are widely known. By way of example, the emission emitted by ¹²³I can be detected by a gamma camera.

In another particular embodiment, the detectable reagent is a fluorophore and detection is performed by detecting the fluorescence emitted by the fluorophore. The use of a fluorophore requires the prior excitation thereof with a wavelength within its excitation spectrum, which causes an emission at a different wavelength. The excitation and emission wavelengths of the fluorophores contemplated in the present invention are part of the state of the art. The emitted fluorescence can be detected, for example, by fluorimetry techniques using a fluorescence spectrophotometer or a fluorescence microscope.

In another particular embodiment, the detectable reagent is a protein. This can be detected depending on the type of protein used. For example:
- an enzyme requires the addition of the specific substrate thereof that will be detectable by colorimetry, chemiluminescence, or fluorimetry;
- a fluorescent protein, like a fluorophore, requires excitation at a suitable wavelength to be detectable by fluorimetry
- an antigen or hapten requires an antibody or other molecule that specifically recognises it. In order to be detected, said antibody or molecule specific for the antigen/hapten must be labelled, for example, with an enzyme, and detection will depend on the type of labelling.

The person skilled in the art will recognise that the third step of the first *in vitro* method of the invention can be carried out as part of detection techniques such as ELONA, ELASA, precipitation and qPCR, gel mobility shift assay, Western blotting, surface plasmon resonance, kinetic capillary electrophoresis, fluorescence binding assay, aptahistochemistry, aptacytochemistry, fluorescence microscopy, or flow cytometry. All these techniques have been explained in the preceding paragraphs of this description, and said explanations are applicable to the present inventive aspect. In a particular embodiment, OR2AT4 detection is performed by means of fluorescence.

In the case where the third step of the first method of the invention comprises the detection of the complex of the invention bound to the OR2AT4 receptor present in the sample, the detection of the complex according to the invention can be carried out with practically any known antibody or reagent that binds with high affinity to the aptamer of the invention or to the functional group. The detection of the aptamer of the invention has been described in detail in the preceding paragraphs. Likewise, in relation to the functional group, detection can also be carried out with virtually any known antibody or reagent that binds with high affinity to said functional group. For this reason, it is particularly suitable for the functional group to be a detectable reagent.

The person skilled in the art will recognise that the third step of the first *in vitro* method of the invention can be carried out as part of detection techniques such as ELONA, ELASA, precipitation and qPCR, gel mobility shift assay, Western blotting, surface plasmon resonance, kinetic capillary electrophoresis, fluorescence binding assay, aptahistochemistry, aptacytochemistry, fluorescence microscopy, or flow cytometry. All these techniques have been explained in the preceding paragraphs of this description, and said explanations are applicable to the present inventive aspect. In a particular embodiment, OR2AT4 detection is performed by means of fluorescence.

In another aspect, the present invention relates to an *in vitro* method for activating the OR2AT4 receptor in a biological sample isolated from a subject, hereinafter, "method for activation of the invention", which comprises contacting said sample comprising the OR2AT4 receptor with the aptamer of the invention, the complex of invention, and/or the combination of the invention.

In a particular embodiment of the method for activation of the invention, the OR2AT4 receptor in the sample is comprised in living cells.

The term "suitable conditions for activating the OR2AT4 receptor", in the context of the present invention, refers to the incubation conditions that allow the binding of the aptamer or complex of the invention to the OR2AT4 receptor and the subsequent activation thereof. These conditions include the composition of the buffer in which the aptamer or complex of the invention is applied to the sample, the amount of aptamer or complex, incubation time, and incubation temperature. Non-limiting examples of buffers suitable for allowing the binding of the aptamer of the invention to the OR2AT4 receptor and the subsequent activation thereof include, but are not limited to, PBS, TBS, phosphate buffer, and citrate buffer. Preferably, these buffers contain 1 mM MgCl₂. the amount of aptamer or complex of the invention necessary to detect the OR2AT4 receptor molecules present in the sample will depend on both the size of the sample and the amount of OR2AT4 receptor molecules present therein, and can be easily determined by optimisation methods that are common in the art. The concentrations of the aptamer or complex of the invention have been described above in the present description for other inventive aspects. The aptamer is incubated with the sample at a suitable temperature and for a time sufficient to allow the binding of the aptamer or complex of the invention to OR2AT4 receptor molecules that may be present in the sample. The temperature is, preferably, between 20°C and 37°C, more preferably 37°C. As a guide, the aptamer will be incubated with the sample for at least 5 minutes, at least 10 minutes, at least 15 minutes, at least 20 minutes, at least 30 minutes, at least 60 minutes, at least 120 minutes, or more.

In a particular embodiment of the method for activation of the invention, the subject is a human. In another particular embodiment of the method for activation of the invention, the biological sample is blood, plasma, serum, urine, cerebrospinal fluid (CSF), heart, brain, or epithelial tissue.

The authors of the present invention have been able to demonstrate the capacity of the aptamer of the invention to inhibit the proliferation of cancer cells and improve the proliferation of skin cells.

Therefore, in another aspect, the present invention relates to the aptamer of the invention, the complex of invention, the combination of the invention, or the composition of the invention for use as a medicament.

In the present invention, the use as a medicament can also be formulated as:
- use of the aptamer of the invention, the complex of invention, the combination of the invention, or the composition of the invention in the manufacture of a medicament.

Another aspect of the present invention relates to the aptamer of the invention, the complex of invention, the combination of the invention, or the pharmaceutical composition of the invention, for use thereof in the treatment and/or prevention of diseases caused by a decrease in the expression of the OR2AT4 receptor and/or by a decrease in the activation of the OR2AT4 receptor and/or by the absence of the natural ligand of the OR2AT4 receptor, and/or a low amount of the natural ligand of the OR2AT4 receptor with respect to the amount of natural ligand in a subject in normal health conditions or a healthy subject. Alternatively, the use of this paragraph can be formulated as:
- Use of the aptamer of the invention, the complex of the invention, the combination of the invention, or the pharmaceutical composition of the invention, in the manufacture of a mediciament for the treatment and/or prevention of diseases caused by a decrease in the expression of the OR2AT4 receptor and/or by a decrease in the activation of the OR2AT4 receptor and/or by the absence of the natural ligand of the OR2AT4 receptor, and/or a low amount of the natural ligand of the OR2AT4 receptor with respect to the amount of natural ligand in a subject in normal health conditions or a healthy subject; or
- Method for the treatment and/or prevention of diseases caused by a decrease in the expression of the OR2AT4 receptor and/or by a decrease in the activation of the OR2AT4 receptor and/or by the absence of the natural ligand of the OR2AT4 receptor, and/or a low amount of the natural ligand of the OR2AT4 receptor with respect to the amount of natural ligand in a subject in normal health conditions or a healthy subject, wherein said method comprises administering to a subject the aptamer of the invention, the complex of invention, the combination of the invention, or the pharmaceutical composition of the invention.

In the present invention, it is understood that an individual/subject is "in normal health conditions" or is "healthy" when the individual does not exhibit symptoms, sign, or indication that he/she suffers from a disease or is ill.

In the present invention, "treatment" or "therapy" is understood as clinical intervention in an attempt to cure, delay, reduce the severity of, or improve one or more symptoms of the pathology characterised by a decrease or inhibition of the expression of the OR2AT4 receptor, and/or a decrease or blockage in the activation of the OR2AT4 receptor, and/or an absence/low amount of the natural ligand of the OR2AT4 receptor (low amount with respect to the amount of natural ligand under normal health conditions). In the present invention, "prophylaxis" or "prevention" or "prevent" is understood as the action of preventing the development or progress of a disease or pathology such as that indicated above.

In a particular embodiment, the disease is selected from the group consisting of: cancer, alopecia, skin wounds, and dermatoses (preferably dermatoses caused by dysbiosis).

Therefore, in another aspect the present invention relates to the aptamer of the invention, the complex of invention, the combination of the invention, or the pharmaceutical composition of the invention, for use in the treatment and/or prevention of cancer in a subject, hereinafter "use for treating cancer of the invention". The use for treating cancer of the invention can also be formulated as:
- Use of the aptamer of the invention, the complex of invention, the combination of the invention, or the pharmaceutical composition of the invention, in the manufacture of a medicament for the treatment and/or prevention of cancer in a subject; or
- Method for the treatment and/or prevention of cancer which comprises administering to a subject the aptamer of the invention, the complex of invention, the combination of the invention, or the pharmaceutical composition of the invention.

In a preferred embodiment of the use for treating cancer of the invention, the cancer is leukaemia; preferably chronic or acute myeloid leukemia.

Another aspect of the present invention relates to the aptamer of the invention, the complex of invention, the combination of the invention, or the pharmaceutical composition of the invention, for use in the treatment and/or prevention of hair loss in a subject and/or in the promotion of hair growth, hereinafter "use for hair of the invention".

The use for hair of the invention can also be formulated as:
- Use of the aptamer of the invention, the complex of invention, the combination of the invention, or the pharmaceutical composition of the invention, in the manufacture of a medicament for the treatment and/or prevention of hair loss in a subject and/or the promotion of hair growth; or
- Method for the treatment and/or prevention of hair loss and/or the promotion of hair growth which comprises administering to a subject the aptamer of the invention, the complex of invention, the combination of the invention, or the pharmaceutical composition of the invention.

In the present invention, the term "hair" refers to the hair found on the scalp of a subject. Therefore, the term "hair loss" refers to the process by which hair from the scalp is lost, temporarily or definitively.

In the present invention, hair loss in a subject preferably refers to accelerated hair loss in a subject resulting in an accelerated deterioration of the hair condition such as: increase in the speed of the hair cycle, decrease in the number of hairs in one and the same hair follicle, decrease in the thickness of hair on the scalp, loss of density of the hair group on the scalp, or appearance of areas on the scalp where there is no hair.

Preferably, in the present invention, the term "treatment and/or prevention of hair loss" refers to the inhibition and/or delay of the hair loss process, or improvement of hair condition, in a subject. Improvement of hair condition in a subject refers to an increase in the thickening of hairs on the scalp, an increase in the elongation of hairs on the scalp, an increase in the number of hairs per square centimetre of scalp surface, and/or an increase in the number of hairs in one and the same hair follicle. Therefore, the treatment and/or prevention of hair loss in a subject results in an improvement in the balance of hair loss/growth, which translates into a larger number of hairs on the scalp.

In the present invention, an increase in the thickening of hairs on the scalp, an increase in the elongation of hairs on the scalp, and/or an increase in the number of hairs in one and the same hair follicle refers to an increase of 1.2 times, 1.5 times, 1.7 times, 2 times, 2.5 times, 3 times, 4 times, 5 times, or 10 times of the number of hairs per square centimetre of scalp surface, the thickening of the hairs on the scalp, the elongation of the hairs on the scalp, and/or the number of hairs in one and the same hair follicle, with respect to a subject who is not administered any treatment to improve the hair condition.

In the present invention, hair loss is preferably caused by one of the following conditions/pathologies: androgenic alopecia, anagen effluvia (for example, anagen effluvium caused by chemotherapy treatment), telogen effluvium (for example, postpartum telogen effluvium), alopecia areata, or inflammatory scarring alopecia.

In another aspect, the present invention relates to the aptamer of the invention, the complex of invention, the combination of the invention, or the pharmaceutical composition of the invention, for use in the treatment of wounds, hereinafter "use for the treatment of wounds of the invention".

The use for the treatment of wounds of the invention can also be formulated as:
- Use of the aptamer of the invention, the complex of invention, the combination of the invention, or the pharmaceutical composition of the invention, in the manufacture of a medicament for the treatment of wounds in a subject; or
- Method for the treatment of wounds which comprises administering to a subject the aptamer of the invention, the complex of invention, the combination of the invention, or the pharmaceutical composition of the invention.

In the context of the present invention, "treatment of a wound" is understood as the acceleration of the healing process of a wound. Non-limiting examples of wounds are:
- a burn wound, which is the injury resulting from exposure to heat, electricity, radiation (for example, sunburn and laser surgery), or caustic chemicals;
- grade 1, 2, 3, and 4 ulcers (classification based on their extent), or pressure, venous, arterial, mixed, iatrogenic, diabetic, and neoplastic/oncological ulcers (classification according to their origin); mouth ulcers, corneal ulcers, genital ulcers, skin ulcers, peptic ulcers (such as gastric ulcer and duodenal ulcer, among others) (classification according to their location).
wounds in diabetes mellitus, which are usually foot injuries due to numbness caused by nerve damage (diabetic neuropathy) and decreased blood flow to the legs and feet. The most serious injury is a foot ulcer. Diabetic foot ulcers have a very high risk of being infected, and sometimes they cannot be cured. Foot ulcers that have not healed are a common cause of amputation in people with diabetes,
- bedsores, in other words, injuries caused by unrelieved pressure on any part of the body, particularly portions on bone or cartilaginous areas,
- wounds due to an external force that damages the tissue,
- wounds on the skin due to ageing or the environment. This includes, for example, cracks, dry Skin, roughness of the skin, and the like.
- wounds on the skin caused by epidermolysis bullosa.

Wounds can be an internal wound, an oral wound, a skin wound, an external wound, an ulcer, and/or bedsore, damage to the eye, a wound in the eye, for example, conjunctiva. Other conditions that can be treated with the aptamers according to the disclosure include oral ulcers, oral aphthous lesions, glossodynia, burning tongue, psoriasis, eczema, and hair loss.

These types of skin damage and lesions are known to the persons skilled in the art. An internal wound is a wound present in the body, for example, due to a surgical incision. An oral wound is a wound present in the oral cavity. A skin wound is a wound present on the skin. An external wound should be understood as a wound that is visible and accessible from outside the body. An ulcer is a lesion on the surface of the skin or a mucosal surface. A bedsore is a wound or ulcer caused by prolonged pressure on the skin and tissues when one is in a particular position for a long period of time, such as lying in bed. The bony areas of the body are the most frequently affected sites, becoming ischaemic under sustained and constant pressure. Skin ageing is the change in the appearance of the skin due to time or exposure to the environment or the state of health of an individual. Cellulitis is the definition used in cosmetics in relation to a flaccid or pitted appearance of the skin (orange peel in Dutch).

In a particular embodiment of the invention, the aptamer, the complex, the combination, or the composition of the invention is used for the treatment of a skin wound. Skin wounds can be wounds in the epidermis or dermis of the skin. There are several types of wounds in which the skin or tissue may need repair: abrasions, lacerations, incisions, perforations and avulsions and burns. In another particular embodiment of the invention, the aptamer, the complex, or the composition of the invention is used for the treatment of an oral wound. Oral wounds are wounds in any part of the oral cavity in which the oral mucosa is damaged. In another particular embodiment of the invention, the aptamer, the complex, or the composition of the invention is used for the treatment of an internal wound. particularly a wound of the internal mucosa. Internal wounds are wounds in which cell layers of endodermal or mesodermal origin are damaged. Examples include wounds in arteries or veins, peritoneum, or pericardium.

The present inventors observed an induction of the expression of antimicrobial peptides in keratinocytes and hair follicle (Figure 12) mediated by OR2AT4 agonist aptamers, which is of particular interest in maintaining and/or restoring the balance of the skin's microbial population.

Therefore, in another aspect the present invention relates to the aptamer of the invention, the complex of invention, the combination of the invention, or the pharmaceutical composition of the invention, for use in the treatment of skin dysbiosis in a subject, hereinafter "use for the treatment of dysbiosis".

In the present invention, the term "skin dysbiosis" or "cutaneous dysbiosis", used interchangeably, refers to the imbalance of the population of microorganisms, preferably bacteria, on the skin. The imbalance of the population of microorganisms on the skin can result in an overgrowth of opportunistic bacteria or colonisation by pathogenic bacteria. Skin dysbiosis on a subject can be caused by external factors such as the colonisation of pathogenic bacteria, or by internal factors such as a defect in the subject's innate immunity or as consequences of a skin disease. Symptoms associated with skin dysbiosis include tightness, itching, and/or burning.

The use for the treatment of dysbiosis can be formulated as:
- Use of the aptamer of the invention, the complex of invention, the combination of the invention, or the pharmaceutical composition of the invention, in the manufacture of a medicament for the treatment of skin dysbiosis on a subject; or
- Method for the treatment of skin dysbiosis which comprises administering to a subject the aptamer of the invention, the complex of invention, the combination of the invention, or the pharmaceutical composition of the invention.

In another aspect, the present invention relates to the non-therapeutic cosmetic use of the aptamer of the invention, the complex of invention, the combination of the invention, or the pharmaceutical composition of the invention, hereinafter cosmetic use of the invention.

In preferred embodiments, in the use for activation of the invention, use as a medicament of the invention, use for hair of the invention, use for the treatment of wounds of the invention, and cosmetic use of the invention, the aptamer of the invention, the combination of the invention, or the composition of the invention is applied topically.

### DESCRIPTION OF THE FIGURES

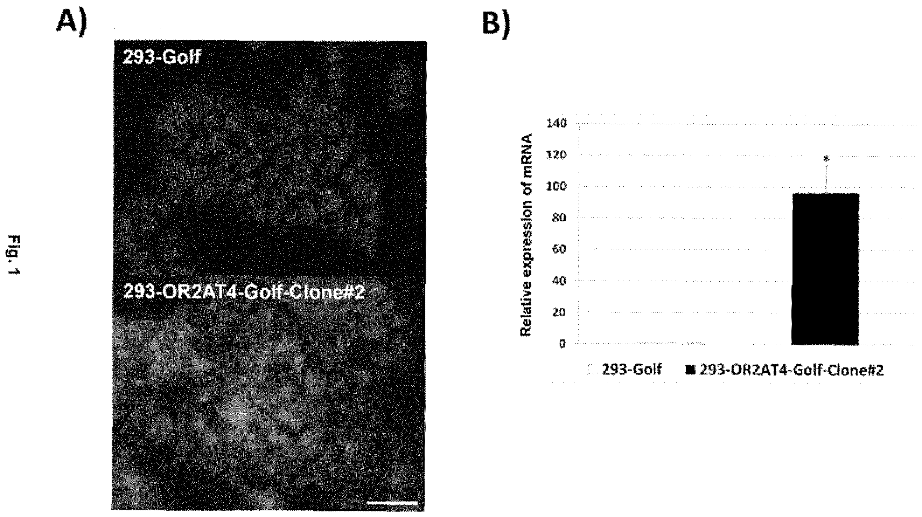

**Figure 1****. Generation of a 293 clone that stably expresses the OR2AT4 receptor.** The 293-OR2AT4-Golf transfectant was generated by means of calcium phosphate cotransfection into 293 cells of the expression plasmids for the OR2AT4 receptor (NM_001005285, pCMV6-Entry-OR2AT4-Neo; Origene) and for the Golf subunit of G proteins, generated by means of subcloning the hGNAL gene (NM_001261443.1) into the expression plasmid (pcDNA3.1-Hygro, GeneScript). Stable transfectants were selected on medium containing neomycin and hygromycin B, and clone 293-OR2AT4-Golf-clone#2 was obtained by limiting dilution. A) Immunofluorescence assay using a polyclonal anti-OR2AT4 antibody that recognises the intracellular domain of the receptor (ThermoFisher Scientific, Waltham, MA, USA) in 293-OR2AT4-Golf-clone#2 cells fixed with paraformaldehyde and permeabilised with Tx-100. Scale: 50 µm. B) The total RNA of 293-Golf and 293-OR2AT4-Golf-clone#2 cells was extracted with the miRNeasy Mini kit (Qiagen, Hilden, Germany) and used to generate cDNA (high capacity reverse transcription system from Applied Biosystems, Foster City, CA). RT reaction products were used as template for amplification by qPCR (Power Sybr Green PCR master mix, Applied Biosystems). Oligonucleotides specific for OR2AT4 and the following amplification conditions were used: 95°C, 10 minutes followed by 40 cycles of 15 seconds at 95°C and 1 minutes at 60°C. The reference gene TATA-binding protein (TBP) was used for normalisation. Replicates of the reactions for the target and housekeeping genes, which were performed simultaneously for each template cDNA analysed, were included in triplicate. Method 2^{-ΔΔCT} was used to calculate the relative expression of each target gene. The control group used for comparisons were 293-Golf cells. The results are depicted as the relative expression of mRNA (fold change) compared to control samples after normalisation with the reference gene TBP.
**Figure 2****. Sequence and structure of the aptamers specific for OR2AT4. A**) The sequence and structure of the aptamers Ap.OR2AT4.7 (SEQ ID NO: 1) and Ap.OR2AT4.17 (SEQ ID NO: 2), obtained by means of Cell-SELEX. **B)** The sequence and structure of the aptamers Ap.OR2AT4.17-45 (SEQ ID NO: 3), Ap.OR2AT4.17-19 (SEQ ID NO: 4), containing a sequence with 45 and 19 nucleotides, respectively, obtained from the 76-nucleotide sequence corresponding to the aptamerAp.OR2AT4.17 shown in A), are shown. C) The sequence and structure of the aptamer Ap.OR2AT4.7-40 (SEQ ID NO: 8), containing a sequence with 40 nucleotides, obtained from the 76-nucleotide sequence corresponding to the aptamer Ap.OR2AT4.7 shown in A), are shown.
**Figure 3****. Activation of cAMP production in 293 cells mediated by OR2AT4 agonist aptamers.** A) 293 cells stably expressing OR2AT4 were transiently transfected with CRE-luciferase and Renilla-luciferase reporter constructs. After 24 hours of transfection, cells were then stimulated with the synthetic odorants Sandalore and Brahmanol (400µM) (A), or with the aptamers Ap.OR2AT4.7 (SEQ ID NO: 1) and Ap.OR2AT4.17 (SEQ ID NO: 2) (10 µM) and the synthetic odorant Sandalore at a suboptimal concentration (200 µM), alone or in combination **(B),** for 4 hours. Data normalisation was calculated by means of the formula [Luc/*Renilla*(*N*)-Luc/*Renilla*(lowest)]/[Luc/*Renilla*(highest)-Luc/*Renilla*(lowest)]. The bar graph represents the normalised luciferase activity for each condition. Means were calculated using three replicates per condition, n=3. Error bars represent the standard deviation, SD. Statistical significance was determined using Student's t test (*p<0.05 and **p<0.001, with respect to control). A representative experiment is shown.
**Figure 4****. Inhibition of the proliferation of the K562 cell line mediated by OR2AT4 agonist aptamers.** The anti proliferative effect of the aptamers on the K562 cell line was evaluated in a colorimetric proliferation assay based on the reduction of tetrazolium salts (XTT). To that end, cells were seeded in 96-well plates and kept in culture in the absence or presence of different concentrations of the OR2AT4 aptamers (1 µM, 10 µM, and 20 µM) for 5 days. XTT assays were performed using samples in triplicate in at least three independent experiments. A representative experiment is shown. The values shown correspond to the means of triplicates and the error bars represent the standard deviation.

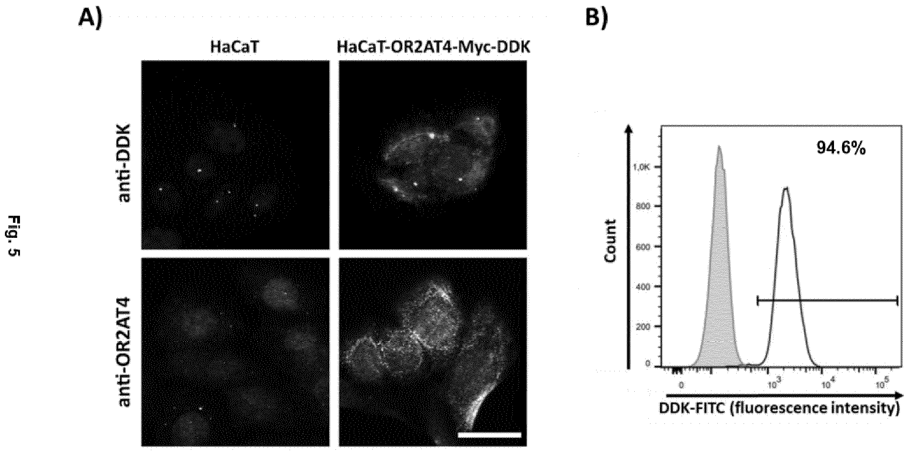

**Figure 5****. Generation of stable OR2AT4 transfectants in the HaCaT cell line.** The human keratinocyte cell line HaCaT was transduced with a lentiviral vector (pLenti-GIII-CMV-GFP-2A-Puro lentiviral vector; Abm Inc.) containing the OR2AT4 sequence fused to a Myc-DDK tag sequence at the C-terminus. **A)** Immunofluorescence analysis in cells fixed with paraformaldehyde and permeabilised with Tx-100, using a rabbit polyclonal anti-OR2AT4 antibody that recognises the intracellular domain of the receptor (left panels) and a monoclonal antibody that recognises the Myc-DDK motif (right panels). Scale: 50 µm. **B)** Flow cytometric analysis of OR2AT4 expression in HaCaT cell transfectants. The expression profile of OR2AT4 in HaCaT-OR2AT4-Myc-DDK transfectants (white histogram) and in the HaCaT parental line (grey histogram) is shown. To that end, the cells were fixed, permeabilised, and stained with an anti-DDK antibody labelled with fluorescein isothiocyanate (FITC).

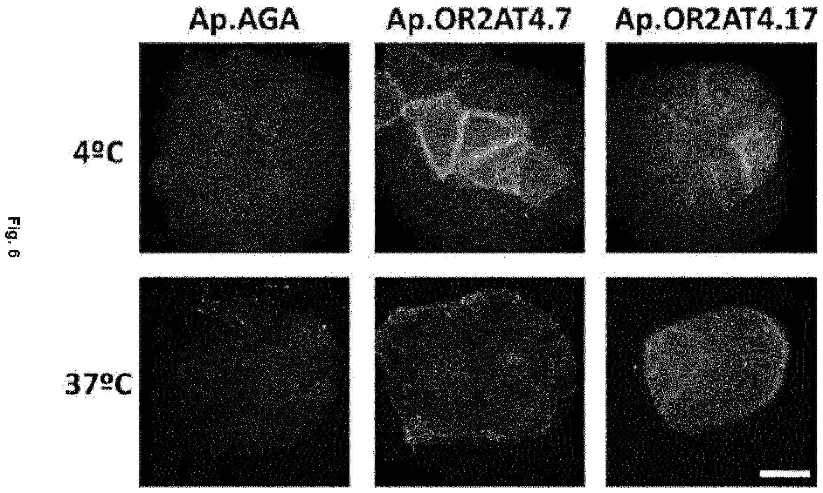

**Figure 6****. Specific binding of the aptamers to the OR2AT4 receptor in HaCaT cells.** HaCaT-OR2AT4-Myc-DDK cells were seeded on 12 mm coverslips and incubated with 15 pmol of the indicated aptamers labelled with the fluorophore AF594, for 45 minutes at 4°C (top panels) or 37°C (bottom panels). The non-specific aptamer 38x(AG) (Ap.AGA) was used as a control. The fluorescence images show the nuclei (DAPI) in blue and the specific signal of the aptamers in red. Scale = 50 µm. The images are representative of 3 independent experiments.
**Figure 7****. OR2AT4 agonist aptamers induce proliferation in HaCaT-OR2AT4-Myc-DDK transfectants.** Cells were seeded in 96-well plates (2,500 cells per well) in DMEM with 10% fetal bovine serum (FBS). After 24 hours, they were deprived of serum (0.5% FBS) for additional 24 hours and the following stimuli were then added in triplicate (day 0): 250 µM Sandalore and Brahmanol **(A),;** 1 µM aptamers (Ap.AGA and Ap.OR2AT4.17) **(B).** Cell proliferation was determined in the HaCaT parental cell line and in the HaCaT-OR2AT4-Myc-DDK transfectants at the indicated times (1, 3, and 5 days) using a colorimetric assay (XTT Cell Proliferation Kit II, Sigma, St. Louis, MO). Both synthetic odorants and OR2AT4 agonist aptamers were capable of inducing a significant proliferative response in the HaCaT-OR2AT4.Myc-DDK transfectants compared to stimulation with the vehicle (Control) **(A)** or with the control aptamer Ap.AGA **(B).** Statistical significance was determined by means of two-way ANOVA and the Bonferroni multiple comparison test (***p < 0.001). Only significant differences with respect to controls are shown.
**Figure 8****. OR2AT4 agonist aptamers induce migration in HaCaT-OR2AT4-Myc-DDK transfectants.** HaCaT-OR2AT4-Myc-DDK transfectants were plated to confluence in 6-well plates and deprived of serum (0.5% FBS) for 24 hours. Cells were then removed from half the well using a cell-scrapper to that end. At this time (t=0), photographs were taken along the entire wound (n=10-12 photographs per well). Aptamers were added at a concentration of 100 nM in DMEM containing 0.5% FBS, and photographs were taken in the same positions as at time 0, after 24 hours, 48 hours, and 72 hours. The graph depicts the areas covered by the cells at 72 hours. The data represents mean±SD (n=3 independent experiments, 10 fields per condition). Statistical analysis was performed by means of two-way ANOVA and Tukey's multiple comparison test (*p<0.05, ****p<0.0001).
**Figure 9****. The aptamer Ap.OR2AT4.17 prolongs the anagen phase of the hair growth cycle and induces hair shaft elongation. A)** Hair follicles (HF) were obtained from the occipital area of the scalp of hair transplant patients. Isolated follicles in the anagen phase were cultured in Williams E medium (Gibco, USA) at 37°C and in a 5% CO₂ atmosphere. After 24 hours, HFs were stimulated with the following conditions: control aptamer Ap.AGA (1 µM), Ap.OR2AT4.17 (1 µM), and vehicle (PBS1×/1 mM MgCl₂). The cultures were restimulated on day 3 and maintained until day 7. To classify the follicles into the phases of the cycle, a morphometric analysis was performed on day 7. **A)** The graph shows the percentage of hair follicles that are in the anagen or catagen phase. The data represents mean ± SEM, n = 5 HFs for each condition, from five different donors. Statistical significance was determined by means of two-way ANOVA and Tukey's multiple comparison test (ns: not significant; ****p < 0,0001). **B)** The length of the hair shaft in the cultures is measured daily until day 7 after treatment with the aptamer (1 µM). A) The graph shows the percentage of hair shaft elongation on day 7 compared to day 0 (mean ± SEM, n=25 HFs from 3-5 different donors). Statistical significance was determined by means of two-way ANOVA and Tukey's multiple comparison test (ns: not significant; ****p < 0,0001).
**Figure 10****. The aptamers Ap.OR2AT4.17 (45), Ap.OR2AT4.17 (19), and Ap.OR2AT4.7 (40) induce cAMP production in HaCaT-OR2AT4-Myc-DDK transfectants.** HaCaT-OR2AT4-Myc-DDK transfectants were treated for 1 hour with increasing concentrations of the aptamers Ap.OR2AT4.17 (45) and Ap.OR2AT4.17 (19), corresponding to SEQ ID NO: 3 and SEQ ID NO: 4, respectively, which consist of shortened versions of the aptamer Ap.OR2AT4.17 (SEQ ID NO:2) **(A)** or with increasing concentrations of the aptamer Ap.OR2AT4.7 (40), corresponding to SEQ ID NO: 8 **(B).** In both cases, samples treated with increasing concentrations of the aptamers corresponding to SEQ ID NO: 2 **(A)** and SEQ ID NO: 1 **(B)** were included for comparison. cAMP levels were quantified using the cAMP-Glo Max assay (Promega Madison, Wl). Results are expressed as mean ± SD increase over baseline level (untreated cells) from two independent experiments, with samples in triplicate.
**Figure 11****. The aptamer Ap.OR2AT4.17 exhibits pro-healing activity in a skin-humanised mouse model.**
   Histological evaluation of healing on day 6 in regenerated human skin. The photographs show representative sections of skin tissues stained with H&E. In mice treated with the aptamer Ap.OR2AT4.17, greater re-epithelialisation of the wound area is observed (greater length of the migratory epithelial tongue) compared to control mice treated with Ap.AGA. The wounds analysed for each treatment are represented based on the percentage of re-epithelialisation on day 6 after injury. Each symbol represents an individual mouse. The means of the percentage of re-epithelialisation are represented. Error bars represent the standard deviation. Statistical significance (****) was determined using Student's t test (p<0.0001).
**Figure 12****. The aptamer Ap.OR2AT4.17 induces the expression of the antimicrobial peptide LL37 in cultured keratinocytes and human hair follicles.**
   **A)** Total RNA extracted from HaCaT-OR2AT4-Myc-DDK cells treated for 24 hours with the indicated aptamers (1 µM) was used to generate cDNA. RT reaction products were used for the amplification of LL37 by means of qPCR. The TBP (TATA box-binding protein) gene was used for normalisation procedures. The graph shows the relative mRNA expression levels compared to control samples (without treatment) after normalisation with the reference gene TBP. The data shows the mean of three independent samples ± standard deviation (SD). **B)** Hair follicles (HFs) in anagen phase obtained from the occipital area of the scalp of hair transplant patients were cultured in Williams E medium (Gibco, USA) at 37°C and in a 5% CO₂ atmosphere. After 24 hours, HFs were well stimulated with vehicle (PBS1x/1 mM MgCl₂) or with the aptamer Ap.OR2AT4.17 (1 µM). The cultures were restimulated on day 3 and maintained until day 7. Sections obtained from cryopreserved tissues were used for the detection of LL37 by means of immunofluorescence using a specific monoclonal antibody (sc-166770; Santa Cruz Biotechnology, Santa Cruz, CA). The photographs show representative images and the graph shows the relative quantification of fluorescence in samples obtained from 3 different patients (n=15). The means are represented and the error bars represent the standard deviation. Statistical significance (***) was determined using Student's t test (p<0.001).

### EXAMPLES

### Example 1. Obtaining OR2AT4 receptor agonist aptamers

A stable transfectant was generated in the 293 cell line for the purpose of identifying aptamers specific for OR2AT4 (Figure 1). To that end, the expression plasmids for the OR2AT4 receptor (NM_001005285, pCMV6-Entry-OR2AT4-Neo; Origene) and for the Golf subunit of G proteins, generated by means of subcloning the hGNAL gene (NM_001261443.1) into the expression plasmid (pcDNA3.1-Hygro, GeneScript), were co-transfected into 293 cells by means of the calcium phosphate coprecipitation method. The plasmids used carry expression cassettes for antibiotic resistance genes (G-418 and hygromycin, respectively), so in addition to keeping the cultures in selection medium, cell clones were isolated by limiting dilution in order to generate a cell line having high expression of the receptor and maintaining the phenotype after successive passages in culture. Clone 293-OR2AT4-Golf-clone#2, which stably expresses the receptor, measured by means of immunofluorescence using an antibody specific for the receptor, was therefore isolated and amplified. Likewise, the expression of OR2AT4 receptor mRNA was quantified by means of quantitative real-time PCR (qPCR) with respect to the parental line 293 (Figure 1A).

The total RNA of 293-Golf and 293-OR2AT4-Golf-clone#2 cells extracted with the miRNeasy Mini kit (Qiagen, Hilden, Germany) was used to generate cDNA (high capacity reverse transcription system from Applied Biosystems, Foster City, CA). RT reaction products were used as template for amplification by qPCR (Power Sybr Green PCR master mix, Applied Biosystems). Specific oligonucleotides and the following amplification conditions were used: 95°C, 10 minutes followed by 40 cycles of 15 seconds at 95°C and 1 minutes at 60°C. The reference gene TATA-binding protein (TBP) was used for normalisation. Replicates of the reactions for the target genes and housekeeping genes, which were performed simultaneously for each template cDNA analysed, were included in triplicate. Method 2^{-ΔΔCT} was used to calculate the relative expression of each target gene. The control group used for comparisons were 293-Golf cells. The results are depicted as the relative expression of mRNA (fold change) compared to control samples after normalisation with the reference gene TBP (Figure 1B).

The clone 293-OR2AT4-Golf-clone#2 was used by means of the Cell-SELEX technique together with the parental line 293 to identify aptamers specific for the OR2AT4 receptor. To that end, six rounds of selection were carried out by means of incubating 293-OR2AT4-Golf-clone#2 cells with an RND40 ssDNA library previously folded in selection buffer (PBS + 1 mM MgCl₂), for 1 hour at 37°C (rounds 1-3) or 30 minutes at 37°C (rounds 4-6). After washing with PBS, recovered cells were used as a template for the amplification of aptamers using primers F3 and R3 (1 µM/primer, 250 µM dNTPs, in a final volume of 100 µl). Counterselection steps were performed before rounds 3 and 6 of Cell-SELEX using 293 parental cell line.

When an enriched population of aptamers is obtained after several rounds of selection, aptamers are amplified using 1U Taq DNA polymerase (Biotools) in 50 µl of reaction containing 1xPCR buffer, 125 mM dNTPs, 1 µM F3 primer and 1 µM R3 primer. The dsDNA product with 'A'-overhang ends is cloned into the pGEM-T Easy vector (Promega). Individual clones are sequenced using the primers T7 (SEQ ID NO: 6: TAATACGACTCACTATAGGG) and Sp6 (SEQ ID NO: 5: ATTTAGGTGACACTATAGAA).

Therefore, 26 clones were obtained, and the corresponding sequences were tested for the capacity to bind to target cells. To that end, 293-OR2AT4-Golf-clone#2 cells and parental cells (20,000 cells/well P96) were incubated with the aptamers (100 ng) for 30 minutes at 37°C. The cells were then washed twice with PBS and resuspended in 10 µl of PBS. Recovered aptamers were amplified by qPCR, in a final volume of 20 µl, including 0.25 µM of primers F3 and R3. The amplification conditions were as follows: 95°C/5 min; 25 cycles [95°C/20 s; 57°C/15 s; 72°C/20 s]. The difference between the Ct corresponding to the aptamer bound to the cells overexpressing the target receptor and the Ct corresponding to the aptamer bound to the parental cell not expressing the receptor indicates the affinity of the aptamer for the target. Of the 26 analysed sequences, 8 aptamers that bound with high affinity to the receptor were obtained, of which the 2 that exhibited the highest levels of binding were selected (Ap.OR2AT4.7 with the nucleotide sequence SEQ ID NO: 1, and Ap.OR2AT4.17 with the nucleotide sequence SEQ ID NO: 2). These aptamers were subjected to secondary structure prediction using the mFold software version 3.5 (http://www.unafold.org/) and the QGRS Mapper server (http://bioinformatics.ramapo.edu/QGRS) to predict G-quadruplex structures (Figure 2).

### Example 2. Activation of the OR2AT4 receptor by agonist aptamers

To check the functionality of the selected aptamers against OR2AT4, a typical GPCR assay (CRE-luciferase reporter activity assay) was used. 293 cells stably expressing OR2AT4 were transiently transfected with CRE-luciferase and Renilla-luciferase reporter constructs. After 24 hours of transfection, cells were then stimulated with the synthetic odorants Sandalore and Brahmanol (400 µM) (Figure 3A), or with the aptamers Ap.OR2AT4.7F and Ap.OR2AT4.17F (10 µM) alone or in combination with the synthetic odorant Sandalore at a suboptimal concentration (200 µM) (Figure 3B), for 4 hours. Data normalisation was calculated by means of the formula [Luc/*Renilla*(N)-Luc/*Renilla*(lowest)]/[Luc/*Renilla*(highest)-Luc/*Renilla*(lowest)]. The bar graph represents the normalised luciferase activity for each condition. Means were calculated using three biological replicates per condition, n=3. Error bars represent the standard deviation, SD. Statistical significance was determined using Student's t test (*p<0.05 and **p<0.001, with respect to control). A representative experiment is shown.

In this assay, the synthetic odorants Sandalore and Brahmanol activated cAMP production in clone 293-OR2AT4-Golf-clone#2 transiently transfected with the CRE-luciferase and Renilla-luciferase reporter constructs (Figure 3A). The aptamers Ap.OR2AT4.7 and Ap.OR2AT4.17 were capable of activating cAMP production at a concentration of 10 µM in agonist mode, producing a synergistic response when used as a stimulus in combination with suboptimal concentrations of the synthetic odorant Sandalore (200 µM) (Figure 3B).

### Example 3. Activation of the OR2AT4 receptor by agonist aptamers

Leukemic cell line K562 was used to demonstrate the functional activity of the aptamers in cells expressing the receptor endogenously. The antiproliferative effect of the aptamers on the K562 cell line was evaluated in a colorimetric proliferation assay based on the reduction of tetrazolium salts (XTT). To that end, cells were seeded in 96-well plates and kept in culture in the absence or presence of different concentrations of the OR2AT4 aptamers (1 µM, 10 µM, and 20 µM) for 5 days. XTT assays were performed using samples in triplicate in at least three independent experiments. A representative experiment is shown. The values shown correspond to the means of triplicates and the error bars represent the standard deviation. The aptamers Ap.OR2AT4.7 and Ap.OR2AT4.17, specific for OR2AT4, were capable of inhibiting the proliferation of this cell line in a dose-dependent manner (Figure 4).

### Example 4. Keratinocyte proliferation and migration mediated by the activation of the OR2AT4 receptor by agonist aptamers

To study the activity of the aptamers in human keratinocytes, stable transfectants were generated for the overexpression of OR2AT4 in the HaCaT cell line, using to that end a lentiviral vector containing the OR2AT4 sequence fused to a Myc-DDK tag sequence at the C-terminus (pLenti-GIII-CMV-GFP-2A-Puro lentiviral vector; Abm Inc.).The expression of the receptor was demonstrated by immunofluorescence in permeabilised cells using OR2AT4 receptor and Myc-DDK tag specific antibodies. Likewise, the expression profile of the receptor in the HaCaT-OR2AT4-Myc-DDK transfectants was analysed by means of flow cytometry using to that end an anti-DDK antibody (Figure 5). To demonstrate the specific binding of the aptamers to the receptor overexpressed in HaCaT cells, HaCaT-OR2AT4-Myc-DDK transfectants were incubated in the presence of the aptamers (15 pmol) labelled with AlexaFluor 594 for 1 hour at 4°C or 30 minutes at 37°C. Cells were fixed with 2% paraformaldehyde and mounted on slides using ProLong Diamond Antifade Mountant mounting medium (Thermo Fisher Scientific, Waltham, MA) containing DAPI. The subcellular localisation of the aptamers was evaluated by means of fluorescence microscopy (Figure 6).

Furthermore, in the HaCaT-OR2AT4-Myc-DDK transfectants, the aptamers identified for OR2AT4 demonstrated functional activity since they were capable of inducing both keratinocyte proliferation (Figure 7) and migration (Figure 8).

### Example 5. Effect of OR2AT4 agonist aptamers on hair follicles

Using a 7-day ex *vivo* culture of human hair follicles (HF) from 4 hair transplant patients, the aptamer specific for OR2AT4 (Ap.OR2AT4.17) prolonged the anagen phase of the cycle, compared to the control aptamer Ap.AGA SEQ ID NO: 7 (Figure 9A), and caused an increase in hair shaft elongation (Figure 9B).

SEQ ID NO: 7, nucleotide sequence of the aptamer Ap.AGA:
AGAGAGAGAGAGAGAGAGAGAGAGAGAGAGAGAGAGAG

### Example 6. Activation of the keratinocyte OR2AT4 receptor mediated by agonist aptamers generated from the aptamers Ap.OR2AT4.17 and Ap.OR2AT4.7

HaCaT-OR2AT4-Myc-DDK transfectants were treated for 1 hour with increasing concentrations of the aptamers corresponding to SEQ ID NO: 3 and SEQ ID NO: 4, which consist of shortened versions of SEQ ID NO: 2 (Figure 10A), as well as with increasing concentrations of the aptamer corresponding to SEQ ID NO: 8, which consists of a shortened version of SEQ ID NO: 1 (Figure 10B). cAMP levels were quantified using the cAMP-Glo Max assay (Promega Madison, Wl). Results are expressed as mean ± SD increase over baseline level (untreated cells) from two independent experiments, with samples in triplicate. Aptamers generated from the aptamers Ap.OR2AT4.17 (SEQ ID NO: 2) and Ap.OR2AT4.7 (SEQ ID NO: 1) increased cAMP production in HaCaT-OR2AT4-Myc-DDK transfectants in a dose-dependent manner.

### Example 7. Effect of OR2AT4 agonist aptamers on healing

Healing experiments were carried out in the skin-humanised mouse model. To that end, immunodeficient NMRI nu (Rj:NMRI-Foxn1nu/nu) mice of 6 to 8 weeks of age, that were orthotopically transplanted with equivalents of bioengineered human skin consisting of a fibrin matrix containing fibroblasts as a dermal component and keratinocytes as part of the epidermal component, were used. After 10-12 weeks of transplant, excisional wounds were made on regenerated human skin using a 2 mm biopsy punch. The administration of treatments was carried out by means of implanting osmotic pumps (Alzet, Cupertino, CA) subcutaneously. The dose of the specific aptamer Ap.OR2AT4.17 (SEQ ID NO: 2) and the control aptamer Ap.AGA was 10 µM in all mice. Tissues were collected 6 days after wound generation, fixed in formalin, and processed for subsequent histological analysis. Samples were fully sectioned using a microtome and tissue architecture was then determined in 1 out of 10 sections by means of haematoxylin-eosin staining, following standard histological procedures to that end. The percentage of re-epithelialisation was determined by means of microscopy using a grating to measure the proportion of each wound that had been covered by neoepidermis in relation to the total length of the wound. Therefore, this percentage was calculated through the formula 100x [(wound diameter-epidermal gap)/wound diameter]. Epidermal gap is the distance between two epithelial tongues. Therefore, the centre of the wound was determined in each sample, and the different percentages of re-epithelialisation between the different samples were compared.

In mice treated with the aptamerAp.OR2AT4.17, greater re-epithelialisation of the wound area is observed (greater length of the migratory epithelial tongue) compared to mice treated with the control aptamer Ap.AGA, demonstrating the effect of the aptamer Ap.OR2AT4.17 on healing and demonstrating the usefulness of the aptamer in the treatment of epithelial wounds (Figure 11).

### Example 8. Effect of OR2AT4 agonist aptamers on antimicrobial peptide production

The OR2AT4 agonist aptamer (Ap.OR2AT4.17) caused an increase in the mRNA expression of the antimicrobial peptide LL37 in HaCaT-OR2AT4-Myc-DDK transfectants (Figure 12A).

Using a 7-day ex *vivo* culture of human hair follicles (HFs) from 3 hair transplant patients, the aptamer specific for OR2AT4 (Ap.OR2AT4.17) caused an increase in the production of the antimicrobial peptide LL37, which was detected by means of immunofluorescence techniques (Figure 12B).

## Claims

1. A nucleic acid aptamer or a fragment thereof capable of binding specifically to the OR2AT4 receptor and activating said OR2AT4 receptor, wherein said aptamer comprises a nucleotide sequence with a sequence identity of at least 80% with SEQ ID NO: 1, SEQ ID NO: 2, SEQ ID NO: 3, SEQ ID NO: 4, or SEQ ID NO: 8.

2. The aptamer or a fragment thereof according to claim 1, wherein the nucleotide sequence comprises the sequence SEQ ID NO: 1, SEQ ID NO: 2, SEQ ID NO: 3, SEQ ID NO: 4, or SEQ ID NO: 8.

3. A complex comprising an aptamer or fragment thereof according to claim 1 or 2 and a functional group, preferably wherein the functional group is selected from the list consisting of a detectable reagent, a drug, and a nanoparticle.

4. A combination comprising:
i) the aptamer or a fragment thereof according to claim 1 or 2, or the complex according to claim 3; and
ii) a compound of formula (I): 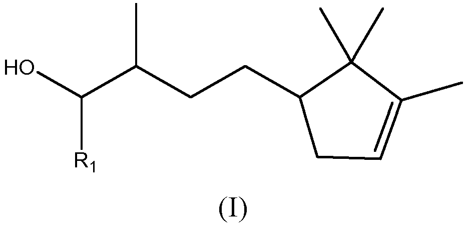
wherein R1 of formula (I) is a hydrogen or a methyl group.

5. A composition comprising the aptamer or a fragment thereof according to claim 1 or 2, the complex according to claim 3, and/or the combination according to claim 4, wherein said composition is a pharmaceutical or cosmetic composition.

6. *In vitro* use of the aptamer or a fragment thereof according to claim 1 or 2, or the complex according to claim 3, for detecting the OR2AT4 receptor.

7. *In vitro* use of the aptamer or a fragment thereof according to claim 1 or 2, the complex according to claim 3, or the combination according to claim 4, for activating the OR2AT4 receptor.

8. An *in vitro* method for the detection of the OR2AT4 receptor in a biological sample isolated from a subject, wherein said method comprises the following steps:
a) contacting the isolated biological sample with the aptamer or a fragment thereof according to claim 1 or 2, or with the complex according to claim 3,
b) separating the aptamer or complex not bound to the OR2AT4 receptor, and
c) detecting the presence of the aptamer or complex bound to the OR2AT4 receptor present in the isolated biological sample.

9. An *in vitro* method for activating the OR2AT4 receptor in a biological sample isolated from a subject, which comprises contacting said sample comprising the OR2AT4 receptor with the aptamer or a fragment thereof according to claim 1 or 2, the complex according to claim 3, and/or the combination according to claim 4.

10. The aptamer or a fragment thereof according to claim 1 or 2, the complex according to claim 3, the combination according to claim 4, or the pharmaceutical composition according to claim 5, for use as a medicament.

11. The aptamer or a fragment thereof according to claim 1 or 2, the complex according to claim 3, the combination according to claim 4, or the pharmaceutical composition according to claim 5, for use in the treatment and/or prevention of diseases caused by a decrease in the expression of the OR2AT4 receptor and/or by a decrease in the activation of the OR2AT4 receptor and/or by the absence of the natural ligand of the OR2AT4 receptor, and/or a low amount of the natural ligand of the OR2AT4 receptor with respect to the amount of natural ligand in a subject in normal health conditions or a healthy subject.

12. The aptamer or a fragment thereof according to claim 1 or 2, the complex according to claim 3, the combination according to claim 4, or the pharmaceutical composition according to claim 5, for use in the treatment and/or prevention of cancer in a subject.

13. The aptamer or a fragment thereof according to claim 1 or 2, the complex according to claim 3, the combination according to claim 4, or the pharmaceutical composition according to claim 5, for use in the treatment and/or prevention of hair loss in a subject and/or in the promotion of hair growth.

14. The aptamer or a fragment thereof according to claim 1 or 2, the complex according to claim 3, the combination according to claim 4, or the pharmaceutical composition according to claim 5, for use in the treatment of wounds.

15. The aptamer or a fragment thereof according to claim 1 or 2, the complex according to claim 3, the combination according to claim 4, or the pharmaceutical composition according to claim 5, for use in the treatment of skin dysbiosis.

16. Non-therapeutic cosmetic use of the aptamer or a fragment thereof according to claim 1 or 2, the complex according to claim 3, the combination according to claim 4, or the pharmaceutical composition according to claim 5.
